# EUROPEAN PATENT APPLICATION

(11) **EP 4 451 286 A2**
(43) Date of publication of application: **23.10.2024**
(21) Application number: 24199554.7
(22) Date of filing: 17.05.2019
(51) Int. Cl.: G16H 50/20

(54) **MANAGING RESPIRATORY CONDITIONS BASED ON SOUNDS OF THE RESPIRATORY SYSTEM**

(30) Priority: 29.05.2018 EA 201800377
(62) Divisional of application: 19727485.5
(71) Applicant: ChestPal Ltd., Brighton BN1 1AE (GB)
(72) Inventor: KARANKEVICH, Aliaksei, Brighton, BN1 1AE (GB); DUBINETSKI, Vitali, Brighton, BN1 1AE (GB); NARUSHEVICH, Yuliya, Brighton, BN1 1AE (GB); ZIABKO, Maksim, Brighton, BN1 1AE (GB); BINETSKAYA, Lena, Brighton, BN1 1AE (GB)
(74) Representative: Fish & Richardson P.C.

(57) **Abstract**

Among other things, sound records captured from a subject by auscultation at sound capture points on the subject are classified among sound classes. Respiratory conditions can be inferred from the sound records and other information. Information about the respiratory conditions can be presented to the subject or to a healthcare provider for purposes of managing the respiratory conditions.

## Description

### Background

This patent application is entitled to the benefit of the priority of the filing date of Eurasian Patent Organization patent application serial number 201800377, filed on May 29, 2018, the entire contents of which are incorporated here by reference.

This description relates, among other things, to managing respiratory conditions based on sounds of the human respiratory system.

### Summary

In general, in an aspect, a sound record is received representing respiratory sounds of a subject acquired by auscultation. By machine, the received sound record is transformed into a time-frequency domain graphical representation. By machine, the time-frequency domain graphical representation is applied to a classifier model to determine a sound class for the respiratory sounds of the subject. By machine, a respiratory condition of the subject is inferred based at least on the sound class determined by the classifier model.

Implementations may include one or a combination of two or more of the following features. The time-frequency domain graphical representation includes a Mel spectrogram. The time-frequency domain graphical representation includes a color Mel spectrogram. The classifier model includes a neural network model. An expert system is used for inferring the respiratory condition of the subject based at least on the sound class determined by the classifier model. The expert system infers the respiratory condition of the subject based also on other information about the subject. The other information about the subject is received from the subject in response to a questionnaire. The other information about the subject includes demographic information. The other information about the subject includes information about a respiratory condition. Information is presented about the inferred respiratory condition through a user interface of a device. The information presented through the user interface includes a graphical representation of the sound record during the period of time. The graphical representation of the sound record is color-coded according to sound class. The information about the inferred respiratory condition presented through the user interface includes information about management of a respiratory condition. Multiple sound records are received that are taken at different sound capture points on the subject. The sound capture points are determined algorithmically based on the respiratory condition, and are presented to the subject through a user interface of a mobile device. Multiple sound records are received that are taken at a particular sound capture point on the subject. A machine performs a principal component analysis or other correlational analysis or multidimensional analysis on the multiple sound records. The sound record has degraded quality. The degraded quality is based on noise or improper auscultation or a combination of them.

In general, in an aspect, a first number of sound records are received. Each of the sound records represents respiratory sounds of a subject acquired by auscultation. Each of the sound records have known sound classes determined by one or more experts. The initial convolutional layers of a neural network are pre-trained using a second number of known spectrograms not necessarily related to sound records. After the pre-training, the initial convolutional layers of the neural network are trained using the first number of sound records and the known sound classes. The second number of sound records are at least an order of magnitude larger than the first number of sound records. A sound record is received for which the sound class has not been determined. The received sound record is applied to the neural network to determine a sound class for the sound record.

Implementations may include one or a combination of two or more of the following features. Operation of the neural network is enhanced by one or more of the following: detecting and eliminating artifacts in the sound records, differentiating different classes of sound records, or adding new sound classes based on new sound records having known sound classes determined by the one or more experts. The neural network includes a truncated model. The truncated model includes a SqueezeNET model. The truncated model is executed on a mobile device. The truncated model is executed on an ARM processor. An expert system uses the determined sound class for the sound record to infer a respiratory condition of the subject. Information is presented about the inferred respiratory condition through a user interface of the device. The applying of the received sound record to the neural network to determine a sound class for the sound record is performed at a server remote from a location where the sound record is captured. The applying of the received sound record to the neural network to determine a sound class for the sound record is performed at a mobile device. The applying of the received sound record to the neural network to determine a sound class for the sound record is performed at a combination of a mobile device and a server remote from the mobile device. The applying of the received sound record to the neural network to determine a sound class for the sound record includes generating a Mel spectrogram for the received sound record. The applying of the received sound record of the neural network to determine a sound class for the sound record includes determining a key-value pair for each of the sound records in which the key includes the sound capture point on the subject and the value includes the sound class. The sound class includes at least one of: normal sound, wheezes, rhonchi, fine crackles, coarse crackles, skin rubbing, interference artifacts, and heartbeat artifacts.

In general, in an aspect, information is received from an application running on a mobile device related to one or more respiratory conditions of a subject. The information includes respiratory sounds captured from the subject by auscultation. The information is processed at the server. Information received from the application running on the mobile device related to the one or more respiratory conditions of the subject is presented to a healthcare provider through a user interface of a device. At the server a determination is received from the healthcare provider about managing the one or more respiratory conditions.

Implementations may include one or a combination of two or more of the following features. The information received from the application running on the mobile device includes information entered by the subject through a user interface on the mobile device. The processing of the information at the server includes applying the respiratory sounds to a classification model to determine sound classes for the respiratory sounds. The processing of the information at the server includes inferring one or more respiratory conditions of the subject. The inferring of the one or more respiratory conditions of the subject is based on the respiratory sounds and on other information received from the subject through the mobile device. The determination of the healthcare provider about managing the one or more respiratory conditions is presented to the subject through the mobile device. The determination about managing the one or more respiratory conditions includes one or more of a diagnosis, a prescription of therapy, training, guidance, or questions. The determination about managing the one or more respiratory conditions includes a binary determination, and the binary determination is presented to the subject through the mobile device. The binary determination presented to the subject includes a determination that the respiratory condition is dangerous or not dangerous, or that the subject should see a doctor or need not see a doctor.

In general, in an aspect, a subject's answers to one or more questions about the subject are received from a mobile device. At a server, the answers are applied to an expert system to infer a respiratory condition of the subject. The expert system infers the respiratory condition of the subject based also on sound records captured by auscultation of the subject.

Implementations may include one or a combination of two or more of the following features. The questions are part of a diagnostic questionnaire or periodic questionnaire. The diagnostic questionnaire or periodic questionnaire relates to a particular respiratory condition. The sound records captured by auscultation of the subject are also received from the device of the subject.

In general, in an aspect, sound records are captured by auscultation at one or more sound capture points on the subject at successive times over a period of time. Based on the sound records captured at the successive times, changes in a respiratory condition of the subject are inferred. Information about the changes in the respiratory condition of the subject are presented through the mobile device.

Implementations may include one or a combination of two or more of the following features. The inferring of changes in the respiratory condition of the subject includes inferring the respiratory condition of the subject at each of the successive times and comparing the inferred respiratory conditions. The inferring of the respiratory condition of the subject at each of the successive times includes classifying at least one of the sound records as representing one or more sound classes. The inferring of the respiratory condition of the subject at each of the successive times includes applying an expert system to the one or more sound classes. The inferring of the changes in the respiratory condition are performed at least in part at the mobile device. The inferring of the changes in the respiratory condition are performed at least in part at a server. The respiratory condition includes a chronic respiratory condition. The chronic respiratory condition includes COPD. The inferred changes in the respiratory condition of the subject include exacerbations. Information about the changes in the respiratory condition of the subject is presented to a healthcare provider through a user interface of a device.

These and other aspects, features, implementations, and advantages (a) can be expressed as methods, apparatus, systems, components, program products, business methods, means or steps for performing functions, and in other ways, and (b) will become apparent from the following description and from the claims.

### Description

Figures 1 and 2 are block diagrams.
Figures 3 through 23 are screen shots of the display screen of a smart phone.

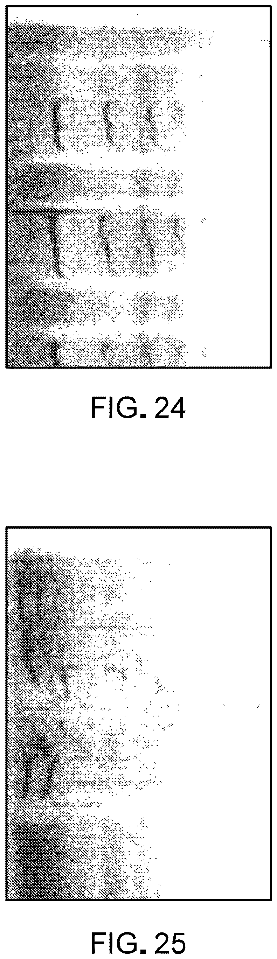

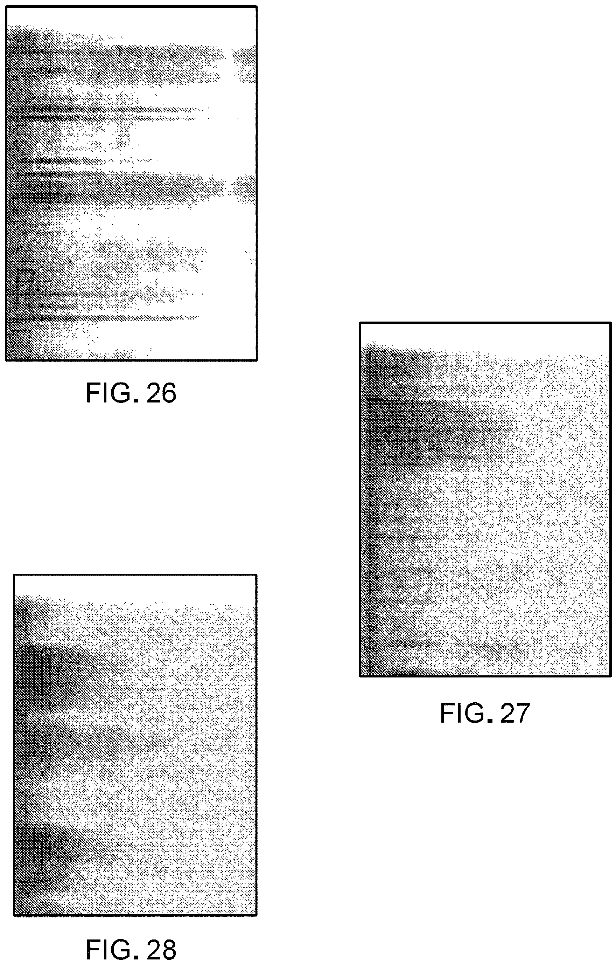

Figures 24 to 28 present examples of spectrograms of the source data for the five classes of lung sounds (wheezes (figure 24), rhonchi (figure 25), fine crackles (figure 27), coarse crackles (figure 26), and normal breathing (figure 28) , respectively).
Figure 29 presents an example of visualization of the signal amplitude-time representation marking.
Figure 30 presents an example of display of the examination result for the doctor.
Figure 31 presents an example of display of the examination result for the patient.

### Introduction

Here we describe technology that we refer to as "respiratory condition technology". This respiratory condition technology is useful for, among other things, sensing, analyzing, characterizing, reporting, and managing conditions of the human respiratory system based on respiratory sounds.

A wide variety of applications are possible for the respiratory condition technology including in medical care, athletic training, parenting, home healthcare activities, and others. In the context of medical care, the respiratory condition technology can be helpful in screening, monitoring, diagnosing, tracking, and managing respiratory conditions such as respiratory diseases that are prevalent in old age, for example, COPD, respiratory conditions in children that are of concern to their parents or caregivers, and other respiratory conditions such as chronic bronchitis, asthma, asthma-COPD overlap syndrome (ACOS), emphysema, lung cancer, cystic fibrosis, pneumonia, idiopathic pulmonary fibrosis, and pleural effusion, to name a few. Although the respiratory condition technology can be useful in screening, monitoring, diagnosing, tracking, and treating respiratory system diseases, it can also be useful for generally understanding, monitoring, and managing other respiratory conditions such as healthy respiratory conditions or differentiating between upper respiratory tract infections and lower respiratory tract infections.

We use the terms "respiratory system" and "respiratory" broadly to include the lungs, the airway, and the respiratory muscles. The airway includes the nose, the mouth, the pharynx, the larynx, the trachea, the bronchi, and the bronchioles and, among other things, provides a passageway for air between the lungs and the outside world.

Respiratory conditions can encompass, reflect, or relate to, among other things, respiratory diseases and respiratory functions. Respiratory functions can include inspiration, expiration, gas exchange, and other functions that relate to the respiratory system's ability to support normal human activity, athletic and other demanding human activity, good health, and other aspects of human life.

Because they play an important role in the quality of life, it is useful to be able to accurately sense, analyze, characterize, screen, monitor, diagnose, report, and manage (we sometimes use the term "manage" broadly to include all such activities and others) respiratory conditions. Traditionally the management of respiratory conditions has been based in part on sensing and analyzing sounds produced by or in the vicinity of the respiratory system in a process known as auscultation. A common device used for auscultation is an acoustic stethoscope which receives respiratory sounds (for example, vibrations within a frequency range of 10 Hz to 25,000 Hz) at auscultation sound capture points on the skin surface of the torso and conducts them acoustically to a part of the stethoscope positioned in or near the ear of the stethoscope user. The user normally has knowledge and experience to interpret the relationship between the sounds heard through the stethoscope and respiratory conditions.

Electronic stethoscopes use electronic techniques to perform at least some of the functions of traditional acoustic stethoscopes. Among other things, electronic stethoscopes can provide respiratory signals representing the respiratory sounds that are picked up at the skin surface. The respiratory signals can be subjected to a variety of analog or digital signal processing techniques aimed at improving the quality of the signals and yielding respiratory sound data representing or derived from the processed respiratory signals. The respiratory sound data can be stored and analyzed using a wide variety of hardware and software to produce information about respiratory conditions.

Respiratory sound data can be associated with different phases of breathing (ventilation), for example, the two main respiratory phases: inspiration and expiration. Respiratory sound data also can be associated with various respiratory sound classes. Respiratory sound classes can in turn be associated with various respiratory conditions. Respiratory sound classes include normal inspiration and expiration (vesicular, bronchial, tracheal), and abnormal classes: wheezing, rhonchi, fine crackles, coarse crackles, and normal breathing.

Respiratory sound data comprises only one kind of respiratory information. Other kinds of respiratory information can be subjective or objective and can include text, image, video, and numeric information about the respiratory system including age, weight, geographic location, medical history, lifestyle, and a variety of other demographic and profile information, as well as answers to questions about the respiratory system of a particular person.

One goal of the respiratory condition technology that we describe here is to automatically or semiautomatically classify respiratory sound data among respiratory sound classes, associate the classified respiratory sound classes with respiratory conditions or exacerbations or improvements in respiratory conditions or both, and then provide information useful to managing respiratory conditions.

Respiratory information, respiratory sound classes, and respiratory conditions of individuals and groups of people are of interest to the individuals themselves (we sometimes call them "subjects"), doctors and other medical care providers, family members, coaches and trainers, and institutions and governments, among others (we sometimes refer to these people and entities as "interested parties"). In addition to being received from and provided to such interested parties, respiratory information generated by and used by the respiratory condition technology can be electronically received from or delivered to and used by mobile devices, tablets, computers, databases, or other electronic equipment for the purpose of analysis, aggregation, storage, and distribution, among other things.

As suggested above, respiratory information about respiratory sound data, respiratory sound classification, and respiratory conditions is useful in a variety of contexts in managing respiratory conditions. In contexts involving medical care, respiratory information can be used as one basis (sometimes in combination with other information) for formally managing respiratory system diseases. In some contexts, respiratory information can be used less formally by individuals and family members, for example, to become aware of, track, understand, and manage conditions, such as the progress of a respiratory virus or the impact of asthma, to name two. Sometimes, respiratory information can be determined and used cooperatively between and among patients, family members, and medical care providers, for example. Other contexts in which information about respiratory conditions may be useful include athletic activities and the impact of weather and other environmental influences, among others. The process of managing respiratory conditions can include understanding or being aware of respiratory conditions, providing and administering medicines and other therapies, observation of the subject, reporting and alerting about respiratory conditions, and other steps designed to maintain good respiratory health, stem the decline of respiratory health, and in some cases improve respiratory health, among other things.

In the respiratory condition technology, a variety of respiratory sound sensors can be used to sense and provide respiratory signals including acoustic stethoscopes, electronic stethoscopes, microphones, and other kinds of contact transducers and acoustic transducers.

Respiratory information is both acquired from subjects and presented to interested parties in a variety of ways as functions of the respiratory condition technology. To a great extent, the acquisition and presentation of respiratory information is effected through user interfaces of applications running on smart phones, tablets, other mobile devices, computers, and other user devices that can be considered part of the respiratory condition technology. Respiratory information is communicated through wireless and wired communication networks including, for example, cellular telephone networks and Wi-Fi. Typically, the respiration condition technology will include one or more servers through which respiratory information passes and is stored and processed, as described later.

Parts of the respiratory information, including respiratory sound data, respiratory sound classes, and respiratory conditions, are acquired, analyzed, processed, and presented in components of the respiratory condition technology, which can include servers and user devices, for example. When presented to interested parties, for example through user interfaces of mobile devices and other devices, respiratory information can take any presentation form, including text, sounds, tables, charts, images, graphs, video, and combinations of them.

A key part of the processing performed by the respiratory condition technology is the classification of respiratory sound data into respiratory sound classes and the inference of respiratory conditions based on the respiratory sound data and other respiratory information. A wide variety of techniques can be used for these activities including artificial and convolutional neural networks, predictive modeling and other machine learning methods, expert systems, and others. Some of these techniques rely on sets of validated data examples, such as respiratory sound data records that have been labeled by doctors or other experts to identify respiratory sound classes to which they correctly relate. The quality of the performance of the respiratory condition technology can depend on the number of available correctly labeled respiratory sound data records for various respiratory sound classes.

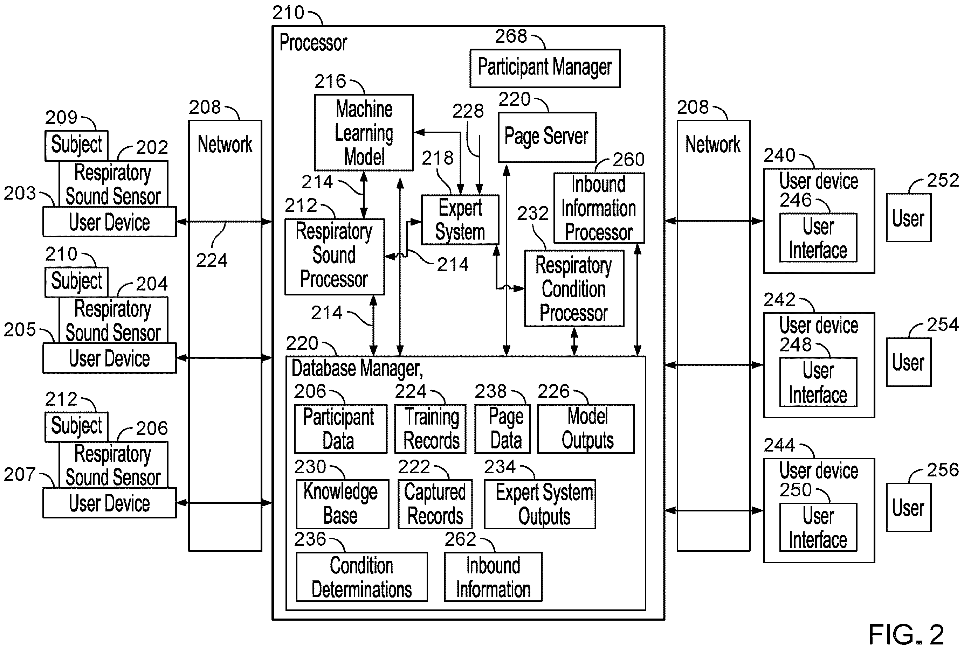

As shown in figure 2, the respiratory condition technology 200 that we describe here includes one or more respiratory sound sensors 202, 204, and 206, each of which can be used with one or more subjects 208, 210, 212. Each of the respiratory sound sensors includes a transducer sensitive to vibrations of sound at or near (e.g., through clothing) a surface of the skin of a subject and is capable of converting the vibrations of the sound directly or indirectly into one or more analog signals. The transducer of each system sound sensor could be, for example, part of an acoustic stethoscope or in electronic stethoscope, a microphone, or another audio transducer, or combinations of them. In some implementations the sound sensors need not be in direct contact with the skin; any device that is capable of detecting respiratory sounds of a subject and converting them to respiratory signals could be used as a respiratory sound sensor.

Each of the respiratory sound sensors can be connected electronically (wirelessly or by wire) or mechanically, or both, to one or more user devices 203, 205, 207. In some cases, the respiratory sound sensor can be part of the user device (such as one or more microphones of a smartphone). In some examples, a cone or other sound conducting device can be situated between such a user device microphone and the skin of the subject to conduct the respiratory sound to the microphone.

As shown in figure 2, the respiratory condition technology also includes a processor 210 that has one or more respiratory sound processors 212 to process the respiratory signals from the sound sensors. The sound processor 212 can perform a variety of signal processing functions including noise reduction using, for example, forward and inverse wavelet transforms, frequency filtering using, for example, a finite impulse response filter (FIR filter), signal normalization, elimination of constant amplitude offset (DC offset), time-frequency representation charting, such as Mel spectrogram calculation, and other techniques, and combinations of them. In general, the sound processor 212 can perform any processing that is necessary or desirable to produce high quality low-noise respiratory sound data 214 in a form useful for later processing steps, including those performed by a machine learning model 216, an expert system 218, and a database manager 220, among others. An example of a sound processor is the audio pre-processing subsystem mentioned later.

Respiratory sound signals can be captured, stored, and processed as digitized sound records 224 each of which has a known start time, a known end time, and a known duration and is associated with a particular sound capture point on the subject. Each sound record can be sensed and processed during a period when a user (say a doctor or a family member or the subject herself) has the respiratory sound sensor positioned to sense the respiratory sounds at that sound capture point.

Each of the captured sound records includes respiratory sound data expressed in a particular digital format and can include sound samples each having an amplitude and a position within a time sequence of sound samples that make up the sound record.

Although figure 2 shows the captured sound records as being sent through the user device and through a network 208 to be processed at the sound processor 212 (which can be in a remote server), the processing of the sound records can be done at the user device or (at least in part) at the sound sensor, or the processing can be split among two or more of the sound sensor, the user device, and the sound processor. The result of such processing of a sound record is a processed sound record 214 ready for further use in the respiratory condition technology. The processed sound record can include digitized versions of the sound samples, metadata about the subject's identity, times of capture, and sound capture points of the sound record, and a color image file of a Mel spectrogram to be used, for example, by a machine learning model 216 for classification purposes.

In addition to being sent to the machine learning model, each processed sound record can be stored as captured sound records 222 in tables of a database 220. The tables of the database can also include respiratory information associated with the captured sound records and entered by the subject or other interested parties through user interfaces of user devices. For example, a subject can enter answers to questions posed by an app running on a mobile device in conjunction with capturing a sound record. Or a doctor can enter findings about a captured sound record.

The machine learning model (e.g., the respiratory sound automatic classification subsystem mentioned later) can receive the sound records either directly from the respiratory sound processor or from the database after they have been stored.

The machine learning model 216 uses known information about other sound records and related respiratory sound classes (such as various classes of wheezing) to classify each incoming captured sound as falling within one or more of the respiratory sound classes. A wide variety of machine learning techniques can be applied by the machine learning model in classifying incoming respiratory sound records. In some cases, the machine learning model can be based on neural networks, for example, artificial neural networks or convolutional neural networks. In some cases, other data comparison techniques can be used to compare an incoming captured sound with known sounds and their corresponding classes to perform the classification.

In some implementations, the machine learning model is trained, before "run time" using a training set of, for example, instances of known sound records that have been correctly labeled by doctors with corresponding respiratory sound classes. Such training records can be obtained as part of the operation of the respiratory condition technology or can be obtained from third parties. In either case, the training records can be stored in training record tables 224 of the database and made available from the database to the machine learning model for training purposes.

The machine learning model is shown in figure 2 as part of the processor 210, but in some implementations, its functions can be performed in the sound sensor, in the user device, or in a combination of two or more of the sound sensor, the user device, and a processor at a server.

The machine learning model can generate, for each incoming captured sound, probabilities for each class; the class with the highest probability is construed as the result of the classification. In some cases, the sound classes are specified with purported certainty. In some instances, instead of specifying definite sound classes the machine learning model can specify probabilities of the correct classes and incorporate those probabilities in the model outputs. The model outputs 226 can be delivered to the database manager for storage and can be delivered directly or indirectly from the stored versions to an expert system 218, among other things.

The expert system 218 applies the model outputs in combination with other respiratory information 228 (and possibly additional information, all together called the "expert system inputs") to an inference engine. The other respiratory information can include a knowledge base 230 stored in the database 220 or in the expert system and retrieved for use in running the inference engine. A wide variety of the other respiratory information in addition to the model outputs and the knowledge base can be used by the expert system including demographic data about subjects, information about geography, weather, time, and other environmental contexts, demographic information about populations of subjects, and information entered by subjects or other interested parties, to name a few. The expert system inputs can be related to a particular moment (the present, for example) and a particular subject and can also include information about other subjects (such as family members or friends of a subject, or populations of subjects, for example, people living in Stockholm, Sweden, or people living in a particular apartment building).

The expert system knowledge base includes information provided by subject matter experts capturing their knowledge about inferences that can be drawn from combinations (either simple or complex) of the expert system inputs. For example, a doctor may provide information that if periods of long duration wheezing are experienced in Siberia during the winter and the subject is a person between the ages of 10 and 15, a good inference is that the respiratory condition is a common chest cold.

The expert system outputs 234 include one or more inferences about the respiratory condition or conditions of one or more subjects or groups or populations of subjects generated by running the inference engine using the expert system inputs and the expert knowledge. In addition to being stored in the database, the expert system outputs 234 are provided to a respiratory condition processor 232 which can use various expert system outputs generated at various times and for various subjects and populations and other respiratory condition information to generate (and store in the database) respiratory condition determinations 236 for use in reporting to subjects and other interested parties. The respiratory condition processor can also generate and store page data 238 associated with the respiratory condition determinations and formatted for use in serving pages to applications and browsers.

The processor 120 includes a page server 220 that serves pages containing respiratory information through the network 208 to user interfaces 246, 248, 250 presented on user devices 240, 242, 244 of users 252, 254, 256. The users can be subjects or other interested parties or combinations of them, and the devices can be the same as or different from the user devices 203, 205, 207. A wide variety of information can be included in the pages served by the page server including information stored in the database and information from other sources (such as external sources of news, weather, demographics, research, articles, statistics, and others). Among the information included in the served pages are questions to be answered by subjects and other interested parties, information about respiratory conditions of subjects and populations of subjects, alerts, charts, advice, guidance, reminders, text, numbers, images, videos, training, historical summaries, statistical summaries, and others, and combinations of them.

In addition to respiration sound records, the processor receives other inbound information at an inbound information processor 260 where it is organized, reformatted, and sent to storage in the inbound information 262 of the database. The inbound information can include answers to questions, images, videos, comments, notes, findings, diagnoses, corrections, instructions, guides, and other information, and combinations of them, including information used by the machine learning model, the expert system model, other components of the processor 210 or server, and the page server.

The subject, interested parties, and other users of the respiratory condition technology can register as users, provide profile information, and manage their accounts by interaction through user interfaces of their devices with a participant manager 268 of the processor 210. The participant manager also can manage security and authentication functions as needed for interactions with user devices. Although shown as part of the processor 210, the work of the participant manager can be shared with processors in user devices. Profile data can be stored in the participant data tables 266 of the database. The profile data is not limited to information provided explicitly by the subject or other interested parties but can also be received from and acquired from third party sources, both public and private. In addition to enabling the maintenance and updating of user accounts, the profile data can be used by the machine learning model, the expert system, the respiratory condition processor, other components of the processor 210 or server, and the page server, and combinations of them.

The processor 210 in figure 2 can be a single processor in a single server, or could be two or more processors located in two or more different servers at two or more locations. The same is true of the database manager 220.

Although many of the uses of the respiratory condition technology involve individual subjects and their specific respiratory conditions, in some implementations, the technology can be used to accumulate, analyse, act on, and report aggregated information about populations of subjects and history of respiratory conditions. For example, the respiratory condition technology can track the development of respiratory conditions over time, across populations, and with respect to geographies to develop information useful in providing warnings and alerts to reduce or limit adverse respiratory conditions affecting populations.

Here we incorporate and supplement the description provided in the Eurasian patent application identified in the first paragraph of this document and provide additional description of the respiratory condition technology.

In some implementations, the respiratory condition technology can include the following components performing at least the following functions with respect to respiratory conditions of the respiratory systems of subjects:
1. A processor (e.g., a signal sampling unit) records respiratory sound signals using a tool for auscultation. The respiratory signals are digitized by pulse code modulation (PCM) and stored in the PCM format by a recording device in the signal sampling unit,
2. A processor (e.g., a questionnaire subsystem) presents questions of a periodic health questionnaire or a diagnostic health questionnaire to subjects and receives and analyses answers to the questions.
3. A processor (e.g., an audio pre-processing subsystem) performs noise suppression, frequency filtering, and frequency-time representation charting by calculating Mel spectrogram,
4. A processor (e.g., a respiratory sound automatic classification subsystem) classifies the respiratory sound using a machine learning model that includes neural network or other machine learning algorithms. The classification model generates values representing probabilities for correctly classifying the respiratory sound in each of the five respiratory sound classes: normal breathing, wheezes, rhonchi, fine crackles, and coarse crackles and in classes of artifacts including: heartbeat, membrane skin rubbing, and interference.
5. In some implementations, a processor (e.g., a respiration phase determination subsystem) segments the respiratory signal into inspiration and expiration phases and time periods when the respiratory sounds of particular respiratory classes (such as wheezing) were recorded, although in some implementations such a step may not be necessary or useful.
6. An expert system (whose inputs are connected to the outputs of the subsystems) determines a respiratory condition based on the respiratory sound classes, the answers to the diagnostic questionnaire and the periodic questionnaire, and the durations of the inspiration and expiration phases,
7. Information about the determinations of the expert system operation are presented through a user interface of a device (e.g., an information output and display unit).

The respiratory condition technology that we describe here is related, among other things, to medical science, specifically to pulmonology, and to the other contexts described earlier. Among other things, the respiratory condition technology is designed to (a) acquire and process respiratory signals, digital versions of respiratory signals, respiratory sound data, and other respiratory condition information, (b) determine respiratory disorders, monitor lung conditions, (c) derive respiratory condition information, and (d) provide respiratory condition information to interested parties for use in a variety of contexts. The respiratory condition technology and the respiratory condition information can be used both at home by a patient (or other subjects) or by a physician at the workplace in a medical institution or via telemedicine, or by any of the other interested parties and in any of the other contexts suggested earlier.

As mentioned earlier, acoustic methods of screening monitoring, and diagnosing changes in human lung based on listening and subjective assessment of the sound phenomena occurring in the lungs-lung auscultation-are well-known and widely used in clinical practice.

Lung auscultation is done by placing a listening device on the chest, for example.

Methods of auscultation of breathing sounds [Russia patent No. 2354285, published on 2009.05.10] and of acoustic diagnosis of focal changes in the human lung-bronchophony [USSR patent No. 1777560, published on 1992.11.23] are known.

A known method and its implementing system for diagnosing respiratory organ diseases [Russia patent No. 2304928, published on 2007.08.27 (the protoclass)] includes the following components connected in series: a signal sampling unit for recording the respiratory sound audio signal using an auscultation tool, an audio signal pre-processing subsystem, a respiratory sounds automatic classification subsystem, a respiration phase determination subsystem based on a breathing activity detection algorithm, a data processing unit, and an information output and display unit. The estimated properties of the respiratory sound conducted to the chest surface are the amplitudes and frequencies of the first (A1, fl), second (A2, f2), and third (A3, f3) spectral maxima located at harmonically related frequencies and having a level of at least 60 dB of the level of the first maximum. Next, the following values are calculated: ratio A12/f21 equal to the ratio (A1-A2) to (f2-f1), ratio A23/f32 equal to the ratio (A2-A3) to (f3-f2), and the difference ΔA12 of the A1 and A2 values between the symmetric survey points on the right (D) and on the left (S) of the subject. The obtained values are then compared to corresponding threshold values for a specific class of disease.

This method allows the diagnosis of pathological focal disorders in human lung by identifying new, more informative, objectively and automatically evaluated properties of the respiratory sounds conducted to the chest surface, which have a high discriminating ability.

The key disadvantage of that method is that any audio signal sampling is sensitive to noise or extraneous actions; for example, if the patient accidentally knocks on the membrane or music plays or people speak loudly near the patient, a wrong signal will be generated, which would make it impossible to extract breathing noises from it.

Other disadvantages of that method are the need for hospitalization and physical examination to implement it due to the lack of a simple personal respiratory health monitoring tool, the absence of a long case history if the person has not visited the hospital for a long time or has visited other hospitals, and inability to remotely view the patient's condition, to listen to their recordings, and warn them about the danger or ask them to visit a doctor.

The new respiratory condition technology that we describe here aims at, among other things, creating a simple, affordable, and robust personal method and system for screening, monitoring, and diagnosing respiratory organs diseases based on ensuring respiratory health monitoring, compiling and maintaining a long case history, providing doctors with the opportunity to remotely view the patient's condition, enabling the patient to listen to the auscultation recordings, and warning the patient about the danger or asking them to make a visit.

These goals have been achieved by the respiratory condition technology that we describe here. In some implementations, this respiratory condition technology provides for respiratory organs diseases diagnosis, which includes the following steps:
1. Using a signal sampling unit for recording the respiratory sound audio signal sensed by a tool for auscultation, an audio signal digitized by pulse code modulation (PCM) is recorded and stored in the PCM format to a recording device in the signal sampling unit,
2. Using a questionnaire subsystem, answers to the questions of a periodic or diagnostic health questionnaire are formed and rated,
3. Using an audio pre-processing subsystem, noise suppression, frequency filtering, and frequency-time representation visualization is performed by calculating Mel spectrum and Mel frequency cepstral coefficients,
4. Using a respiratory sound automatic classification subsystem, classification evaluation of the respiratory sound is carried out using neural network machine learning algorithms, which results in the probability of assigning the respiratory sound to one of the five classes: normal breathing, wheezes, rhonchi, coarse crackles, and fine crackles, for example, and also to classes of artifacts including: heartbeat, membrane skin rubbing, and interference.
5. Using a respiration phase determination subsystem, in some implementations, the source signal is split into inspiration and expiration phases, as well as into the time periods when the characteristic respiratory sound, such as wheezing, was recorded,
6. Using an expert system, final assessment of the patient respiratory health status is generated, taking into account the classification evaluation of the respiratory sound and the answers to the diagnostic and periodic questionnaire questions,

The results of the expert system's operation are presented via an information output and display unit.

In some implementations, the system for screening, monitoring, and diagnosing respiratory organs diseases (containing series-connected signal sampling unit for recording the respiratory sound audio signal with an auscultation tool, an audio signal pre-processing subsystem, a respiratory sounds automatic classification subsystem, a respiration phase determination subsystem based on the voice activity detection algorithm, a data processing unit, and an information output and display unit) successfully achieves the goal because additionally (a) the system is equipped with a questionnaire subsystem, (b) the data processing unit is designed as an expert system, whose inputs are connected to the outputs of all the subsystems, (c) some implementations could include (optionally), a breathing phase determination subsystem is designed to split the original audio signal into inspiration and expiration phases and to mark the time intervals when wheezing was detected, (d) the respiratory sound automatic classification subsystem is operated on the basis of machine learning algorithms with the possibility of obtaining a result in the form of a set of probabilities of correctly assigning the respiratory sound to one of the five classes: normal breathing, wheezes, rhonchi, fine crackles and coarse crackles. and to artifact classes including: heartbeat, membrane skin rubbing, and interference., and (e) applications operating through user devices exposed interactive features and recommendations that provides significant value to the patient.

Machine learning is a well-known technology, which can be used, for example, as described in [USA patent No. 7,149,347, published on 2016.12.12] or-in the technical area of this description-as described in [A. Kandaswamya et al., Neural classification of lung sounds using wavelet coefficients. Computers in Biology and Medicine, 34 (2004) 523-537].

Machine learning, in the respiratory condition technology described here, uses a set of audio signals of respiratory sound previously collected, for example, by physicians from obviously sick people and from healthy people. In some implementations, each of the audio signals can be 5 seconds long, expressed in a pulse code modulation (PCM) 16-bit format, and collected using Android smartphones at a sampling frequency of 44,100 Hz. In some implementations, the sampling frequency need not be so high and could be a sampling frequency in the range of 40 Hz up to 8 kHz. Each of the audio signals represents a lung sound. The initially collected lung sounds are validated by a board of doctors whose goal is to give a qualitative assessment of (e.g., label) each of the sounds by assigning each of the sounds to one of the five respiratory sound classes (normal breathing, wheezes, rhonchi, fine crackles, coarse crackles), for example, thus forming a labeled respiratory (e.g., lung) sound data set of sound records.

In one example, the data set was collected by physicians from the Belorussian hospital, part of a scientific group called Belarusian Medical Academy of Postgraduate Education (BelMAPO). All records of the data set were captured using an Android app on Android smartphones. The only function of the app was to record the lung sounds. Basic physician acoustic stethoscopes were modified by attaching a microphone and connecting the microphone by wires to the smart phone. After the lung sounds were recorded, physicians listened to each record of the dataset. Groups of three physicians reached a consensus decision about the class of lung sound represented by each of the recorded sounds (sound records) of the data set. The size of the data set included 121 records for each of the five different classes to be covered by the training set and 60 records for each of the five different classes in a test set for a total of 905 records in the data set. In some implementations, the data set would have a larger (for example, a much larger) number of records. For example, the data set might include 6000 training records and 3000 test records.

Then, in some implementations, a logarithmic Mel spectrogram is built for each signal, saving the result as a colour (RGB) image in the .PNG format; it is these images (the Mel spectrogram is for the signals of the set, that is, each of the sound records of the set) that serve as an initial training data set (a learning sample) for the machine learning. In some implementations, the number of labeled signals in the training data set is relatively small (for example, fewer than 1000 labeled signals for each sound class). In such cases, due to the limited size of the learning sample, it is not possible to use standard approaches in deep model learning, so it was decided to use a learning transfer approach. Such implementations of the learning transfer approach are based on a convolutional neural network (CNN) as a feature extractor (to extract features from the learning sample for use in assigning signals to classes). In some implementations, the technique could also be based on a recurrent neural network (RNN) ending with one fully connected layer to obtain classification results in the form of a probability of assignment to one of the above classes. That is, the neural network output is a list of probabilities of assignment of an input sample to one of the classes. Later components in the processing sequence can simply use the class having the highest probability.

In some implementations, an Xception model [Xception: Deep Learning with Depthwise Separable Convolutions, Francois Chollet, Google, Inc., http://openaccess.thecvf.com/content cvpr 2017/papers/Chollet Xception Deep Learning CV PR_2017_paper.pdf] is used as a feature extractor (described above as the CNN), while the RNN is implemented on the basis of a long short-term memory model (LSTM). The models use the learning sample to learn sequentially: CNN first, and RNN only after the CNN has been trained. Both models learn using the error backpropagation method. Cross-entropy is used as a loss function, Adam (adaptive moment estimation) is used as the optimization algorithm for RNN, while CNN uses RMSprop [https://towardsdatascience.com/understanding-rmsprop-faster-neural-network-learning-62e116fcf29a] as its optimization algorithm. Thus, the problem of sound recognition (that is, the problem of classifying an unknown sound record) becomes a problem of image (spectrogram) recognition, which is solved using a model originally trained on the ImageNet [http://image-net.org/index] data set (using a small set of spectrogram data found on ImageNet), and additionally trained using the available learning sample data; this is how the learning transfer approach is implemented. When learning is finished, the trained model is tested to determine its accuracy. For testing, 500 records (e.g., stored signals of sounds, e.g., stored sound records) are run through the resulting trained model, and the resulting classes are compared to the standard labels received from the doctors.

Once the trained model has been tested and confirmed to be accurate, the server's trained model (neural network) is permanently loaded into random access memory, and all current requests coming to the server from the outside for classification are processed by the trained model, which outputs the above described result. In some implementations, the expert system part of the respiratory condition technology does not require human intervention; it is called an expert system because it uses the same decision-making methods used by the experts-roughly speaking, because it is built by experts to perform the same actions that expert humans would perform if the expert system were not present.

This invention is presented in the Eurasia patent application in non-limiting drawings:

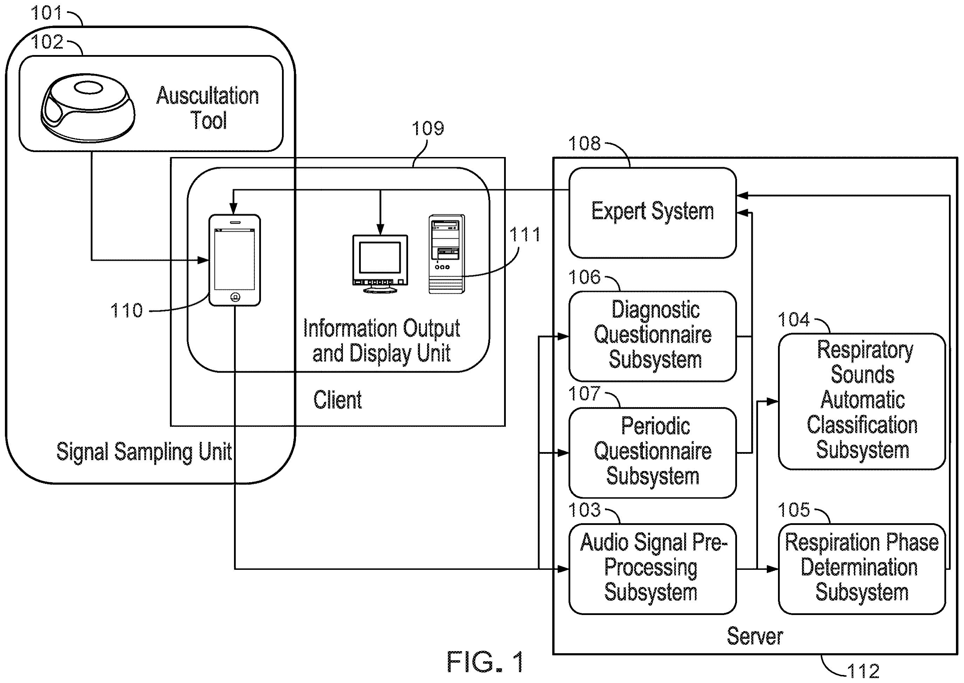

As shown in Figure 1, in some implementations the respiratory condition technology comprises a series-connection of a signal sampling unit 101 for recording the respiratory sound audio signal using an auscultation tool 102 and in some cases a smart phone 110, an audio signal pre-processing subsystem 103, a respiratory sounds automatic classification subsystem 104, a respiration phase determination subsystem 105 (not required) based on a voice activity detection algorithm, and a questionnaire subsystem comprising a diagnostic questionnaire subsystem 106 and a periodic questionnaire subsystem 107. The data processing unit is designed as an expert system 108, whose inputs are connected to the outputs of all subsystems. There is also an information output and display unit 109 (which can include the smart phone 110)whose input is connected to the output of the expert system 108. Some of the components are part of a server 112 as shown.

In some implementations, all processing takes place on a server, the display unit is located on a doctor's PC and on the patient's smartphone, and data acquisition is effected on the smartphone.

In some cases, some or all of the processing can occur on the patient's smartphone. In some instances, the processing occurs on a combination of the server and the patient's smart phone. In some implementations, some of the processing can be done on an electronic stethoscope or similar device.

In some implementations, the auscultation tool 102 can be designed in the form of an electronic stethoscope having a Bluetooth module, which includes an analogue-to-digital converter, a Bluetooth transmitter for transmitting the data to the data processing unit, a battery that provides power to the Bluetooth module, and a microphone amplifier; in some implementations, the auscultation tool can be designed in the form of a stethoscope attachment for a smartphone. A variety of implementations are possible for the auscultation tool including commercially available components. In some instances, the lung sounds are recorded directly using a smartphone microphone.

In some implementations, the audio signal pre-processing subsystem 103 uses algorithms from the signal digital processing field, namely noise reduction using forward and inverse wavelet transforms, frequency filtering using a finite impulse response filter (FIR filter with the cutoff frequency of, for example, 8 kHz), signal normalization (for example, peak normalization) by elimination of constant amplitude offset (DC offset), and time-frequency representation, in particular Mel spectrogram calculation, which is desirable to prepare the input data for the machine learning algorithm. In cases that involve larger data sets, noise suppression filtering steps can be reduced and other alterations made to the sequence of processing steps.

In some implementations, the respiration phase determination subsystem 105 splits the original current audio signal into inspiration and expiration phases and the periods when wheezing was detected. The splitting defines a set of time intervals, with each time interval defined by the start time and the end time of detected inspiration, expiration, or wheezing. The respiration phase determination subsystem is based on all the respiratory sound activity detection algorithm. Selection of input information for this algorithm, such as the upper and lower frequencies in the analysed sound activity is carried out for each audio signal individually. In some implementations, there is no need to split the audio signal into inspiration and expiration phases and periods when wheezing was detected.

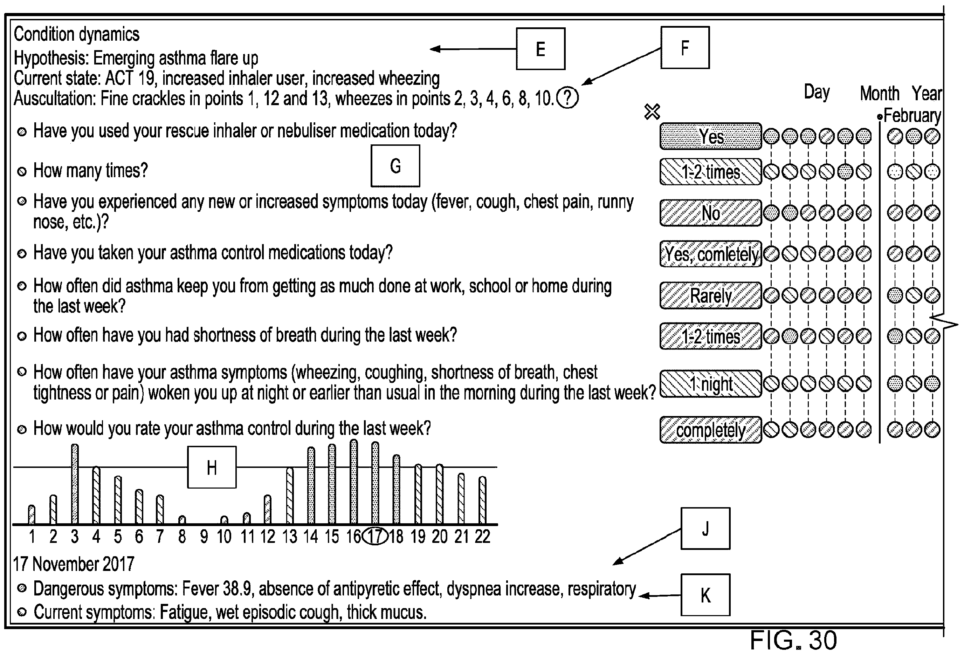

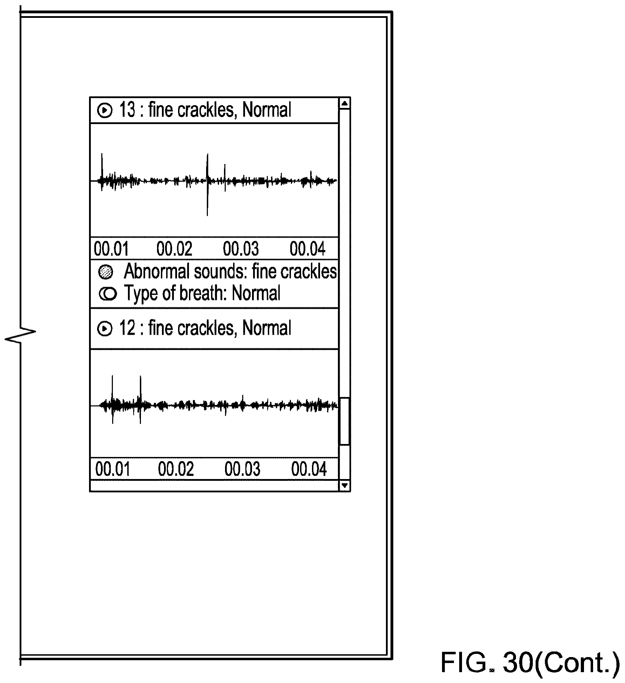

In some cases, the resulting sets of time intervals with breathing phases and wheezes for the lung sounds could be used to visually mark the signal's amplitude-time representation to identify the phases and wheezes for the viewer (This is displayed in the doctor's visualisation unit, for example, and looks approximately as shown in Figure 30.) and to calculate the amplitude and duration of each of the phases. The maximum value in the interval between -20 dB and -40 dB is used as the amplitude. The duration of each time interval is typically between 400 and 5,000 milliseconds (the entire signal can sometimes include only inspiration or expiration). The amplitude and duration could, in some implementations, subsequently be used in the expert system 108.

In some implementations, information can be provided to participants other than a doctor and the patient, and the split of information presented respectively to the doctor and the patient could be different. In some cases, the respiratory condition technology provides an API for use by external telemedicine systems, which can make use of the classification of class of lung sounds and underlying sound recordings in a variety of ways. The telemedicine systems could also perform their own classification using the underlying sound recordings.

In some implementations, the respiratory sound automatic classification subsystem 104 is based on the deep machine learning algorithms and is organized as described above. As described above, the trained model (neural network) is loaded into the random access memory, and all the requests for classification coming to the server from the outside are processed by the trained model, which outputs the classification result in the form of a set of probabilities of correctly assigning the respiratory sound respectively to the five classes: normal breathing, wheezes, rhonchi, fine crackles and coarse crackles, and also the probabilities of artifact classes, including: heartbeat, membrane, skin rubbing, and interference.. Although these five classes of respiratory sound are commonly used and well-recognized, in some implementations, other respiratory sound classes could be classified in use for a variety of purposes. Such other respiratory sound classes could include some kinds of breathing done by children or bronchial breathing and other sound classes mentioned earlier. Additional training records for the data set would need to be created to support such other classes. In some implementations, the goal of the respiratory condition technology is to classify respiratory sounds that suggest COPD and chronic asthma or exacerbation or return to baseline for a disease progression, for example. For those respiratory conditions, wheezing and crackles and normal breathing are important aspects of respiratory sounds to be classified. This classification result is subsequently used by the expert system.

In some implementations, the model can be deployed on the smart phone rather than on a server, or aspects of the model can be executed in a combination of a smart phone and the server.

In some implementations, the diagnostic questionnaire subsystem 106 includes a list of questions proposed to the patient; the results, that is, the answers, are used in the expert system. There are several classes of questions determined by the nature of the permitted answers: single choice answers, a choice among several options, and numeric answers. Each of the answers is assigned its own weight, which affects the final decision made by the expert system. The diagnostic questionnaire is presented to the patient to determine their current condition of respiratory health in order to obtain information suggesting the most probable problems..

In some implementations, the periodic questionnaire subsystem 107 uses a periodic questionnaire that is inherently similar (but in a different context) to the diagnostic questionnaire, except that the periodic questionnaire is offered periodically for continuous monitoring of respiratory health and contains questions aimed at acquiring the constantly changing information for the case of a particular patient, because the set of information for different diseases identified by the patient earlier may differ significantly as time passes. One difference in the context between the periodic questionnaire and the diagnostic questionnaire is that the periodic questionnaire provides patient information useful in understanding the dynamics of the known disease, while the diagnostic questionnaire provides information about what has gone wrong that may indicate possible screens, monitors, and diagnoses.

Examples of periodic questionnaires and diagnostic questionnaires that have been tested and verified are available (either for public use or under license) on the World Wide Web, for example, at https://ccq.nl/?page_id=342., https://catestonline.org, https://mdcalc.com, and https://asthmacontroltest.com and are incorporated here by reference. Periodic questionnaires and diagnostic questionnaires can be configured specifically for users with respect to particular respiratory conditions, such as asthmatics, patients with chronic obstructive pulmonary disease (COPD), for pneumonia, and for bronchitis.

On the clinical side, COPD patients are more likely to use technology continuously during the winter period when there is a high chance of exacerbation. They always experience some symptoms and abnormal lung sounds are always present. Having longitudinal data (data accumulated over time) is paramount for detecting COPD exacerbation early. For asthma it is often the opposite. Those patients with asthma that is not severe are more likely to use technology as a second validation point after peak flow. They typically will not have wheezes in the controlled condition, so presence of wheezes is very indicative. Severe cases of asthma should be monitored like COPD.

In some implementations, the questionnaire format is used for acquiring information because it is analogous to questions that a doctor would ask a patient in a normal examination. In this sense, the questionnaire emulates the doctor's conduct. The use of the questionnaire format also enables differentiation of questions and answers (on different questionnaires) based on different possible respiratory conditions, based on expert suggestions offered by doctors, and in light of differences in profile data for different patients. For example, an asthma patient is asked questions related to her asthma condition and a COPD patient can be asked questions related to her COPD condition. Because classifications made by the system are to be used by doctors, and because the doctors have standard effective questions for patients with different respiratory conditions, the questionnaire format is sensible and effective.

In addition, whether or not the format of information gathering is expressed in a questionnaire or in some other way, it is important and useful to accumulate respiratory condition information in addition to the recorded sounds in order to arrive at clinically significant outcomes and other effective management of respiratory conditions. In some implementations, it may be possible to reduce the scope or entirely eliminate the questionnaires by using other kinds of information such as speech or physical activity of the patient, to name two.

In some implementations, a goal of the respiratory condition technology is to understand two things about a patient based on classification of class of lung sounds (sound classes) or questionnaire results and profile data or both: is the user an otherwise healthy patient who is sick; and for a patient with a chronic disease, has the health condition improved or worsened. The questionnaires provide information useful in screening, monitoring and diagnosing illness and chronic disease.

In some implementations, the expert system's 108 input receives the information from the above subsystems including: the result of respiratory sound automatic classification, in some cases the sets of time intervals identifying the phases of breathing and wheezing, and the answers to the diagnostic questionnaire, the periodic questionnaire, or both questionnaires. The purpose of the expert system 108, among other goals, is to aggregate the information from all the subsystems in order to make a final decision about the most probable problems in respiratory health of the patient and the possible changes in them over time, for better or worse. The processing of all the data is of an expert nature: for each respiratory disease, there are individual decision-making branches for assigning the current data set and other input information to a specific disease; the decision-making branches take account of all possible combinations of answers to the questionnaire and the results of respiratory sound automatic classification.

Although the system is intended to provide recommendations and statistics based on the two sources of information (classes of respiratory condition based on sound recordings and answers to questions on the questionnaires) in some implementations, the recommendations can be based on only one of the sources without the other and in some implementations can also be based on other sources.

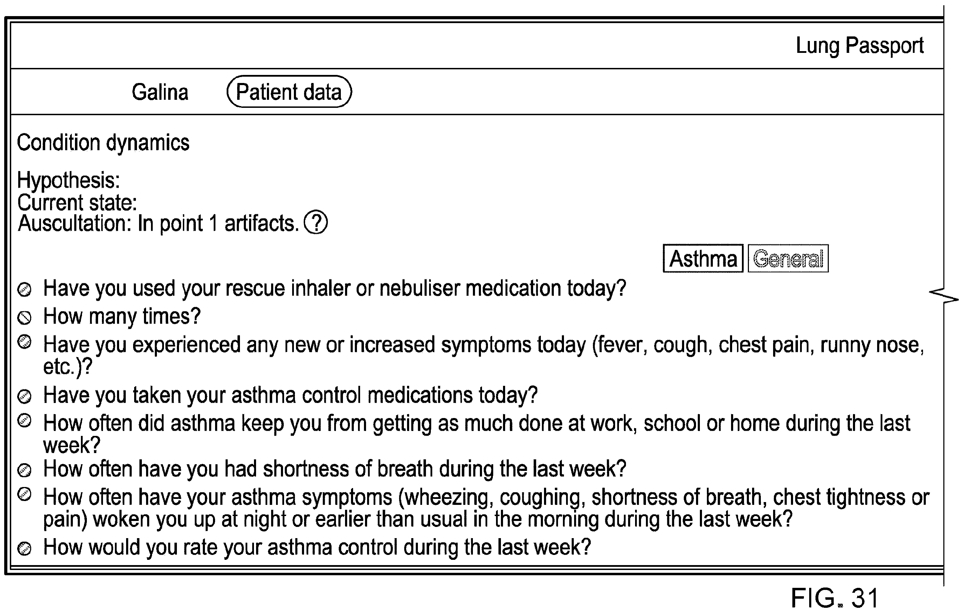

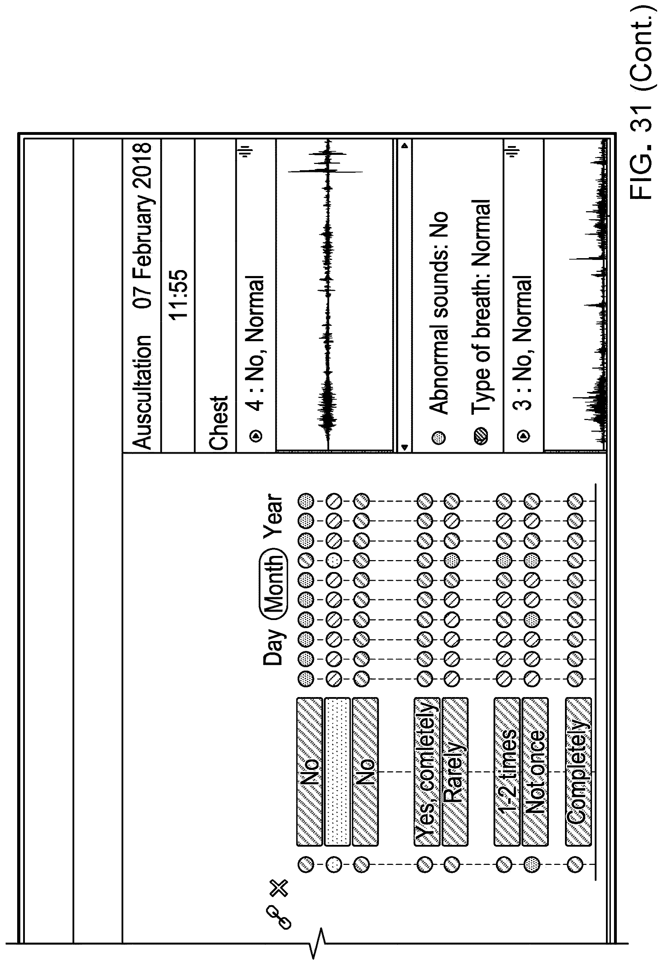

In some implementations, the information output and display unit 109 can be organized into two parts - one for the attending doctor (Figure 30) and one for the patient (Figure 31).

In some implementations, when the respiratory condition technology is used, the patient, for example, records audio signals periodically (for example, once a day or, in exacerbated cases, twice a day, although in some implementations it may be possible to do it less often than once a day or more often than twice a day) using the auscultation tool 102 and gets access to the visualized statistics about their health status, which is implemented in a mobile application developed, for example, on the Android platform and based on the results of the expert system's 108 operation.

A mobile application is used to collect the respiratory sound audio signals from an electronic stethoscope, to enable a user to enter the answers to the diagnostic and periodic questionnaires, and to maintain the statistics. In some implementations it may be possible to receive the peak flow and/or spirometry data automatically from other medical devices. In some implementations, it may also be possible to receive information based on activity tracking. In many basic implementations, the steps are: collect the lung sound data and questionnaire results, classify the lung sounds, process the answers and classification results, and give the patient or other interested parties detailed statistics and just-in-time recommendations to manage respiratory conditions.

These functions are part of what concerns the display unit (an example of the user interface of such a display unit is shown in Figure 31). In the example shown in Figure 31, the displayed statistics for the patient illustrate several zones designated in this example by letters A to D in accordance with the following descriptions: A) Symptom dynamics diagram and maximum expiratory flow rate (peak flow) diagram, B) Results of the automatic analysis of the respiratory sounds including an automatic summary, C) Personalized statistics based on the history of measurements and their results, and D) Automatic recommendation. In some implementations, through the user interface of the display unit, the patient receives a link to their "card" on the website to be able to send it to another doctor, if needed, and receives notifications from the doctor in case of an urgent need to contact them.

In general, the patient has access to the patient's statistics through the user interface of a mobile app. In general, doctor has access to a more comprehensive set of information through a webpage. In some implementations, a read only webpage can be made available for doctors to share information about a patient.

In some implementations, the doctor gets access to the patient's respiratory health statistics (and possibly other information about respiratory condition) maintained on the server through webpages of a remote website displayed through a browser on a PC or on a mobile device, including the ability to organize a communication session between the doctor and the patient, if needed. An example of data received by the doctor is shown in Figure 30. The data is divided into zones, as well, designated in this example by letters E to K in accordance with the following descriptions: E) the neural network's hypothesis about the user's possible illness or other respiratory condition, based on auscultation, the results of the diagnostic questionnaire, and previous respiratory condition history, F) the result of the user's latest auscultation indicating the sound capture points where wheezes were detected, G) the user's answers to a questionnaire (for one selected day), H) the user's condition chart (based on auscultation), J) dangerous symptoms identified by the questionnaire (daily, weekly, or diagnostic) for one selected day, and K) symptoms identified by the questionnaire (daily, weekly, or diagnostic) for one selected day.

In the respiratory system technology, the recorded respiratory sound audio signals are processed respiratory sound with subsequent analysis of the time-frequency properties of the signals, which allows classifying the respiratory sounds and screening, monitoring, and diagnosing the patient's health status.

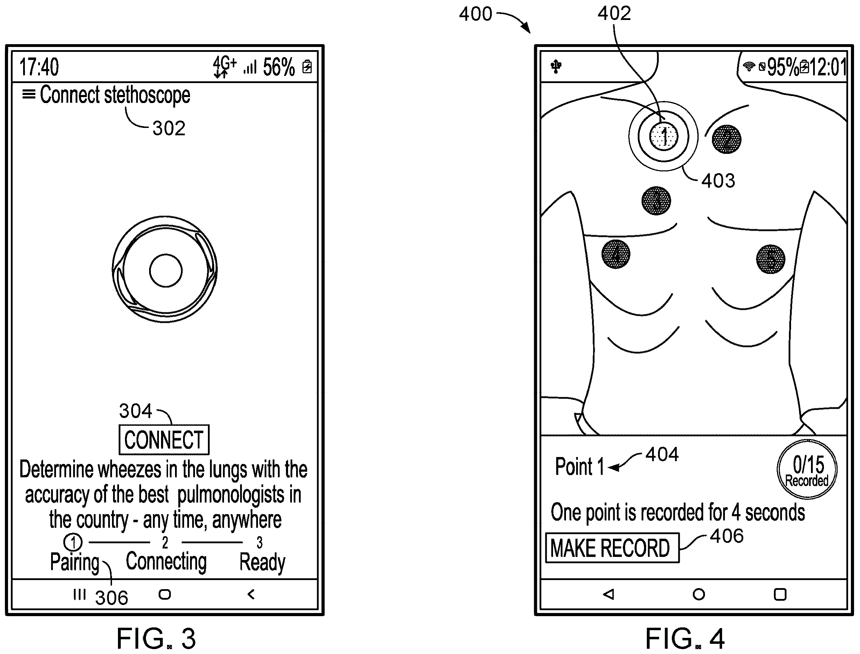

In some implementations the processing of the recorded respiratory sound audio signals is executed as follows: An electronic stethoscope's membrane is tightly applied to the patient's body. The sound capture points coincide with those during typical auscultation performed by the doctor during examinations. The sound capture points are located on the patient's chest, back, and sides to cover all lung areas. The recording conditions include the presence of at least one phase of breathing (the patient's inspiration or expiration) in the audio signal, since it is at these times that respiratory sounds are detected. A chart with sound capture points and a sequence of the sound capture points is displayed in the user interface on the patient's smartphone. Figures 4, 5, and 6 show auscultation sound capture points on the front, side, and rear surfaces of the patient's chest, and these are identified in the display in the user interface. The order of the sound capture point numbers from 1 to 15 corresponds to a preferred sequence of application of auscultation sound capture points. In some implementations, it may be possible to identify and use other auscultation sound capture points based on experimentation. In some cases, the number of auscultation sound capture points can be reduced, for example, when used by healthy people. In some instances, when chronic diseases are involved, more specific or other auscultation sound capture points may be identified and used to improve the quality of the classification process.

Some implementations, the respiratory sound recording at each body application sound capture point occurs according to the same scenario: the electronic stethoscope 102 connects to the patient's smartphone using Bluetooth technology and records a 5-second-long audio signal with 8 kHz sampling frequency (based on the capability of the microphone used in the electronic stethoscope) in pulse-code modulation format, which is saved to the storage device of the patient's smartphone. If a pathological respiratory sound is identified, the patient may be asked to perform auscultation and recording at 2 to 3 points near the point where the pathological respiratory sound was detected; this is helpful to confirm the newly discovered sound. In some implementations, for each 5-second-long audio signal recording, the following actions are performed: the collected audio signal is uploaded to the server, where the classification system decides whether the audio signal represents pathology or not (or more generally determines respiratory condition corresponding to the sound class associated with the audio signal). The server returns the answer (e.g., the determined respiratory condition) to the mobile device, which checks whether it is worthwhile to offer the patient to record additional sound capture points; thus, in such implementations, the sound analysis takes place on the server using a classification system.

In addition to the examples of user interface displays discussed above, figures 3 through 23 illustrate how some implementations of the user interface of an application running on a smart phone of the subject could be arranged.

As shown in figure 3, once a subject has registered with the respiratory condition technology and installed the corresponding application on the subject's smart phone, the subject can begin the process of auscultation. The application instructs the subject to connect with stethoscope 302 and provides about 304 to enable the subject to cause the connection. The smart phone then pairs itself with the wireless stethoscope through a Bluetooth connection. The status of the pairing is illustrated at 306.

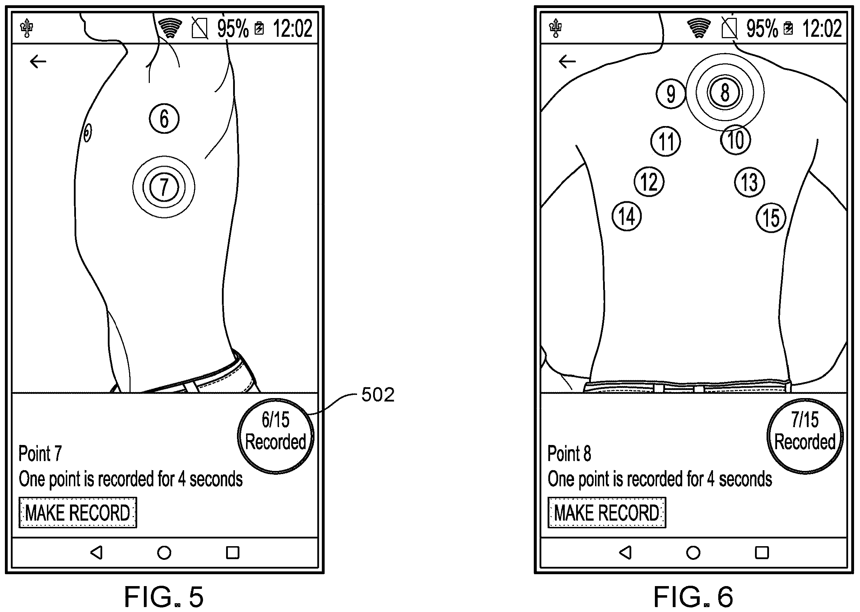

Next, the subject is shown different sides of a body (front, back, and side) on three successive screens 400 (figures 4, 5, and 6). Each side is labeled by numerical labels 402 identifying sound capture points 1 through 15. The smart phone application highlights 403 each of the sound capture points in turn to indicate to the user a sequence in which sound records should be captured at successive sound capture points. In each case, once the subject has placed the stethoscope at the labeled sound capture point, the subject can click the "make record" button 406. The subject holds the stethoscope at that sound capture point for at least four seconds. Progress in the capturing of sound records from point to point is reported to the subject at element 404 and progress indicator 502 (figure 5).

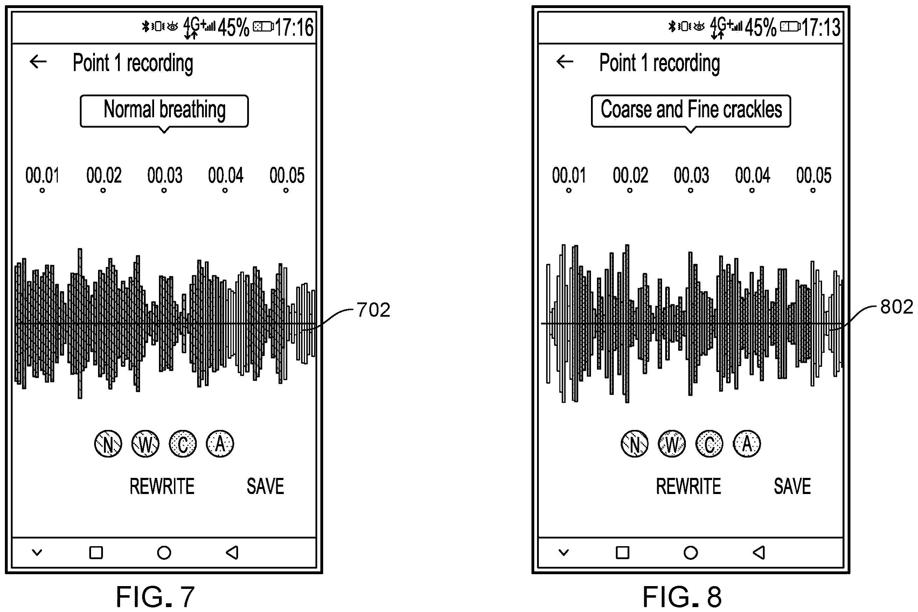

Once a sound record for a particular sound capture point has been captured and processed by the classification model, the application on the smart phone can display the sound record graphically as shown in the middle of figure 7. The graphical representation of the sound record is formed by vertical bars each of which has the magnitude of the sound record for a given sample. Moments in time are identified by dotted time indicators range from left to right above the graphical representation of the sound record.

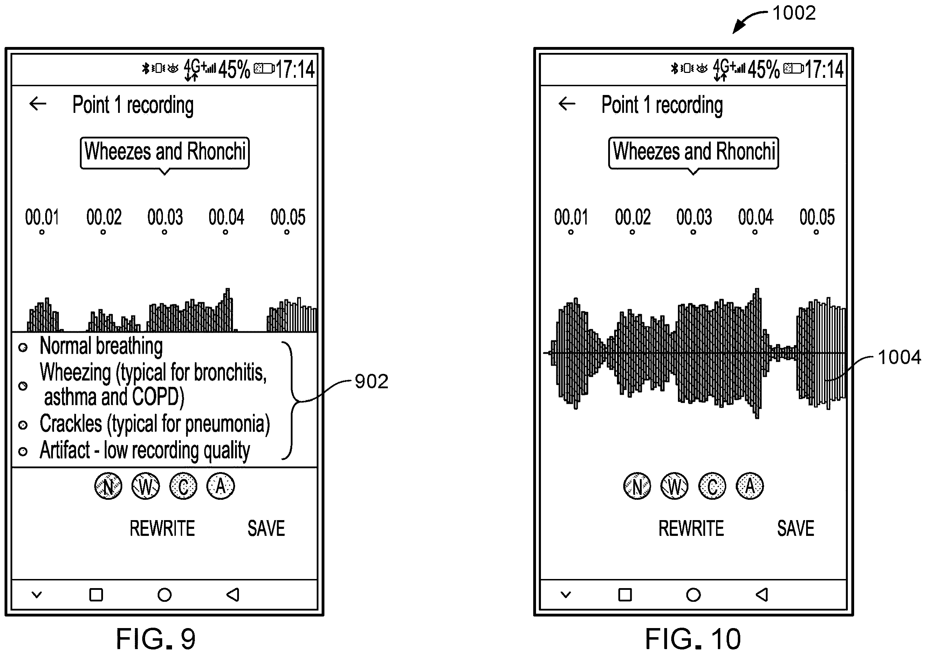

Results of applying the classification model to identify sound classes based on the sound record are indicated by colors or shading applied to the respective sample bars of the graphical representation. In the illustrated example, the classification model identifies four sound classes: normal breathing, wheezing, crackles, and artifacts. Before sound classes are identified by their first letters in four buttons below the graphical representation of the sound record. By clicking on any of the four buttons, the subject is shown the sample bars of the graphical representation at which the classification model identified that sound type. For example, in figure 7, normal breathing was identified in the brightly colored bars 702. In figure 8, coarse and fine crackles were identified in the brightly colored bars 802. In figure 9, wheezes and rhonchi are shown in the brightly colored bars on the right-hand side, and the descriptions of the four sound classes are provided to the subject. Figure 10 shows the wheezes and rhonchi bars 1004 more clearly. Each of the graphical representation displays includes a header 1002 that identifies the sound capture point associated with the graphical representation.

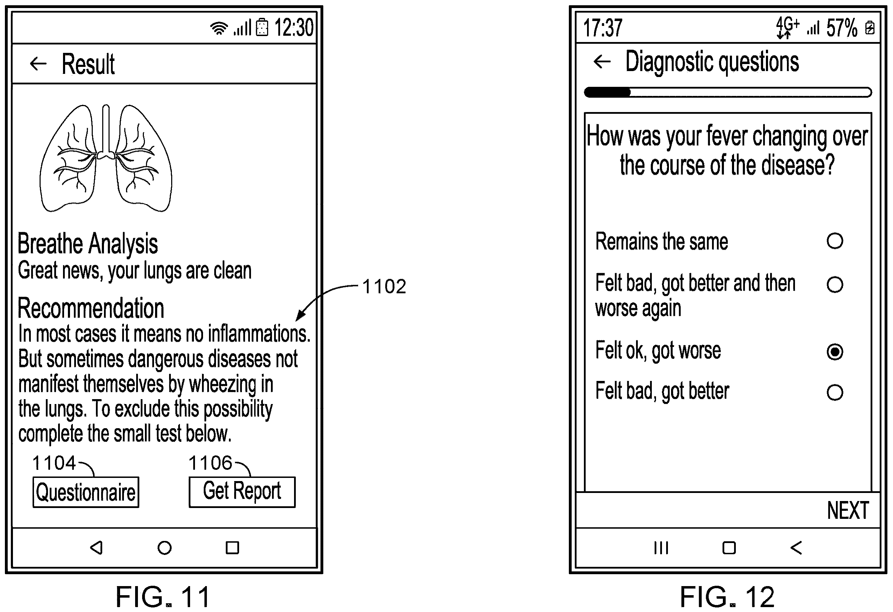

As shown in figure 11, once the subject has finished capturing sound record that all of the indicated sound capture point, a result of the classification process and the application of the expert system to the results of the classification process can be presented in a summary prose format 1102. Two buttons are provided. One button 1106 enables the subject that a full report of the results. The other button 1104 enables the user to proceed with the questionnaire that can be helpful in the operation of the expert system.

Figures 12 through 16 illustrate features of screens presented by the application on the smart phone as part of questionnaires. A diagnostic questionnaire could include questions of the kind illustrated on figures 12 through 16.

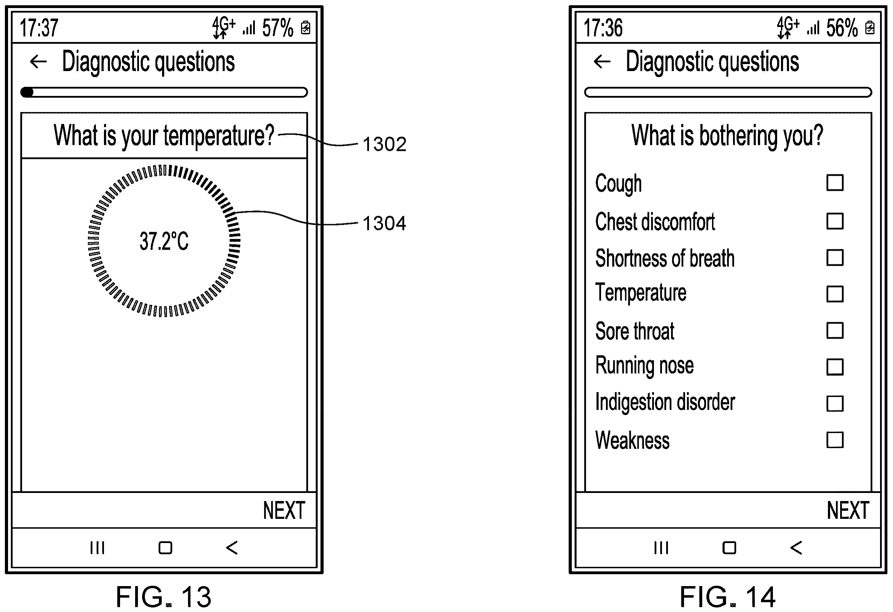

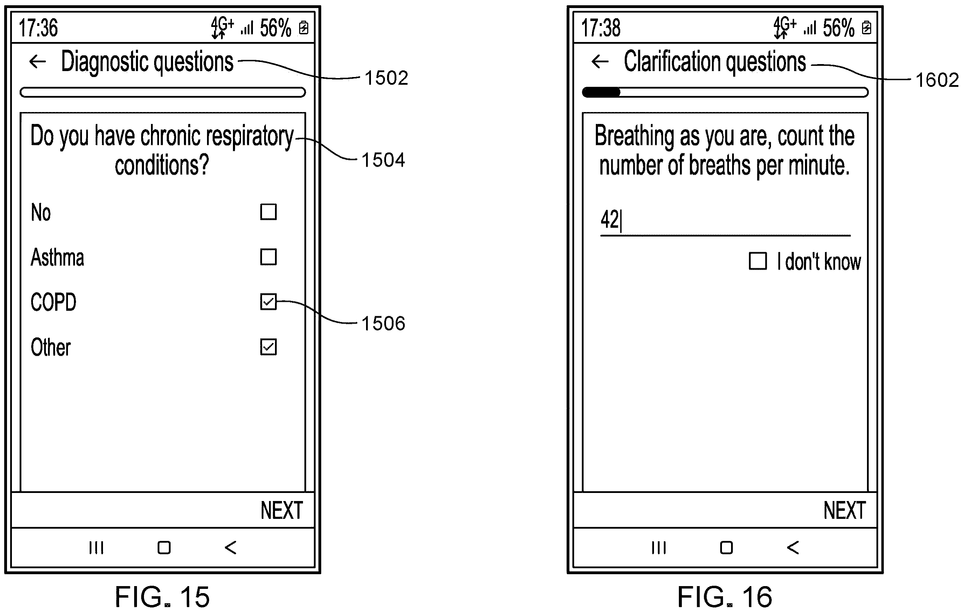

As shown in figure 12, a diagnostic question could ask for information about changes in symptoms. As shown in figure 13, for example, the application can ask 1302 the subject to indicate her current body temperature using a circular indicator 1304. An earlier diagnostic question in the questionnaire, illustrated on figure 14, could ask a very general question about symptoms. Figure 15 illustrates a diagnostic question 1502 that is phrased 1504 to list specific known respiratory conditions that are chronic, asking the subject to identify them by checkmarks 1506. When the application running on the smart phone recognizes the need to ask a clarification question 1602, it can pose the question. As shown, a question that is part of the questionnaire comments for numerical information and request the subject to determine the appropriate number to be inserted as the answer.

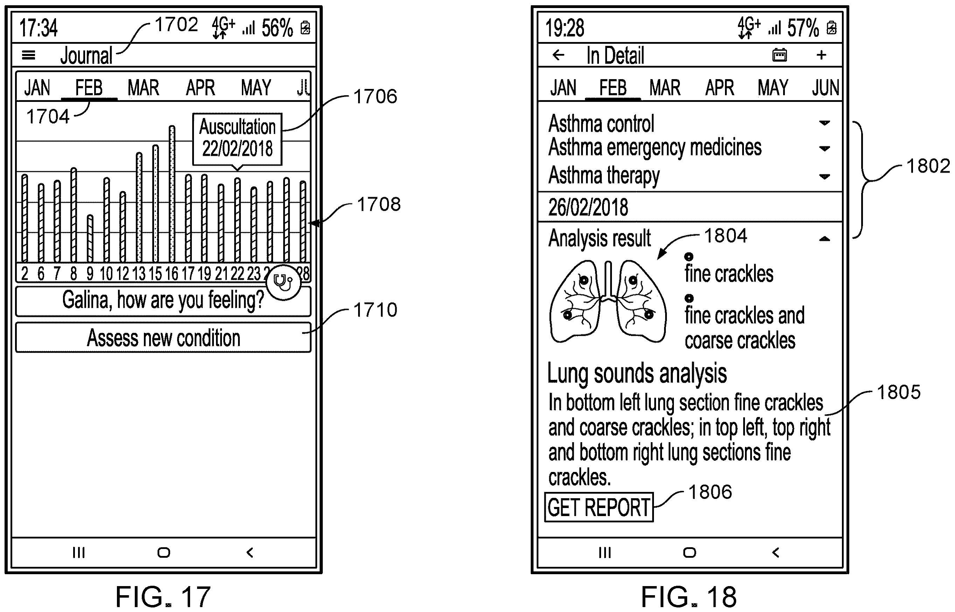

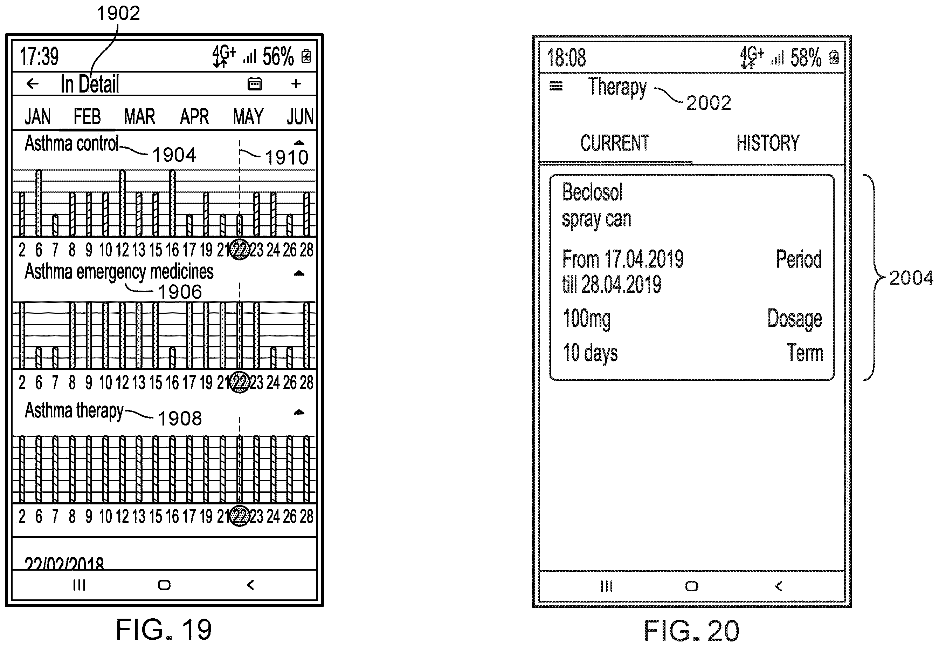

As shown in figure 17, the application running on the smart phone can present a journal showing the history of auscultation sessions 1706 for a given month 1704 in a series of months. A vertical bar for each day of the month illustrates the detected lung sounds. Different colors could be used to indicate different sound classes. For example, a green bar could mean `normal', yellow could mean 'wheeze/rhonchi', and red could mean 'fine/coarse crackles'. The higher the bar, the more widespread the sounds are (by number of recording points where they were detected). In addition to viewing the journal, the subject can click on a button 1710 to enter information about a new condition and have it assessed. Figures 18 and 19 illustrate "in detail" 1902 screens available to the subject to understand more detail about a condition for a given month. In figure 18, the user has four controls 1802 with which to reveal additional information about a particular respiratory condition, in this case, asthma. The fourth button, titled "analysis result" can be invoked to show the results of the application of the respiratory condition technology to the sound records provided by the subject. The schematic illustration of the lungs 1804 is annotated to show where particular sound classes were identified. A prose description 1805 is also included. By clicking on a button 1806, the subject can get additional information in a more detailed report. Figure 19 illustrates the results of opening the links titled "asthma control" 1904, "asthma emergency medicine" 1906, and "asthma therapy" 1908. Vertical line 1910 identifies a particular day under consideration at the moment. The vertical bars in each of the sections of the screen display illustrate successfully completed activities associated with managing the asthma. Figure 20 is a therapy 2002 display providing information 2004 about a particular therapy being used by the subject.

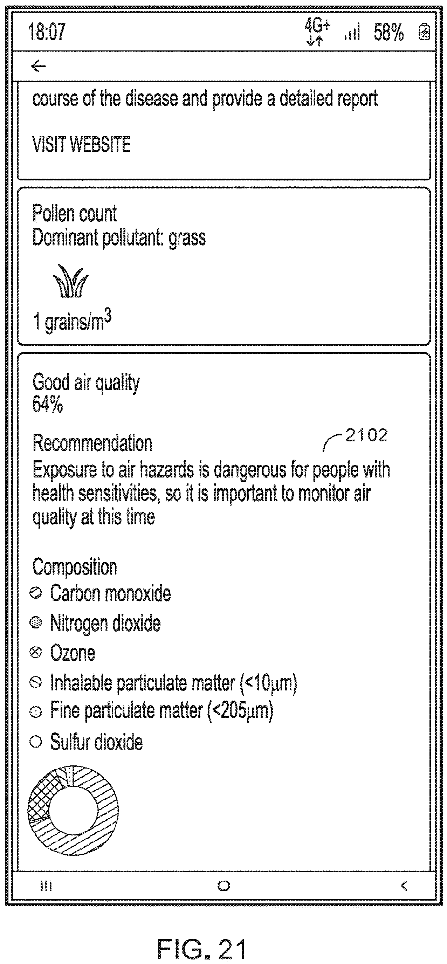

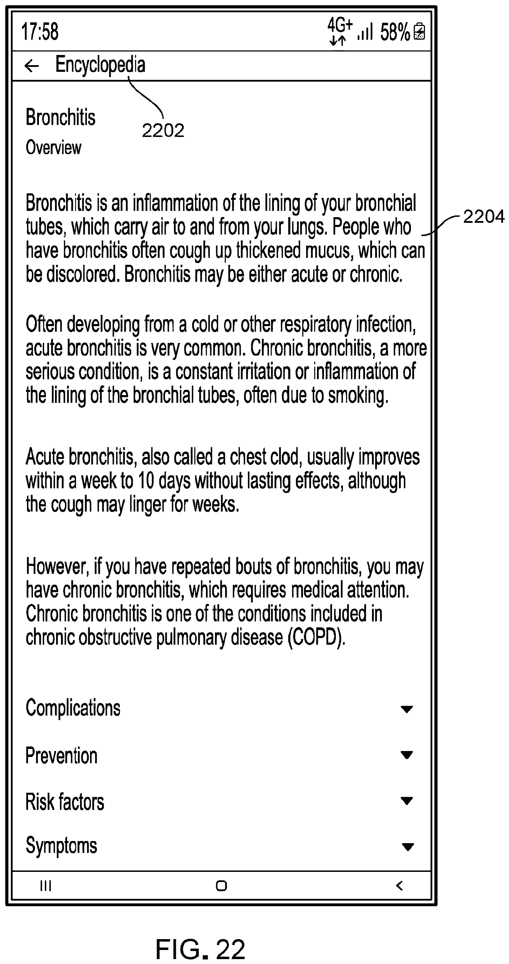

The application running on the smart phone can also provide educational information related to respiratory conditions. As shown in figure 21, for example, a screen can report on air quality and provide a recommendation to the subject. In figure 22, a longer prose explanation 2204 of bronchitis from an encyclopedia 2202 is displayed.

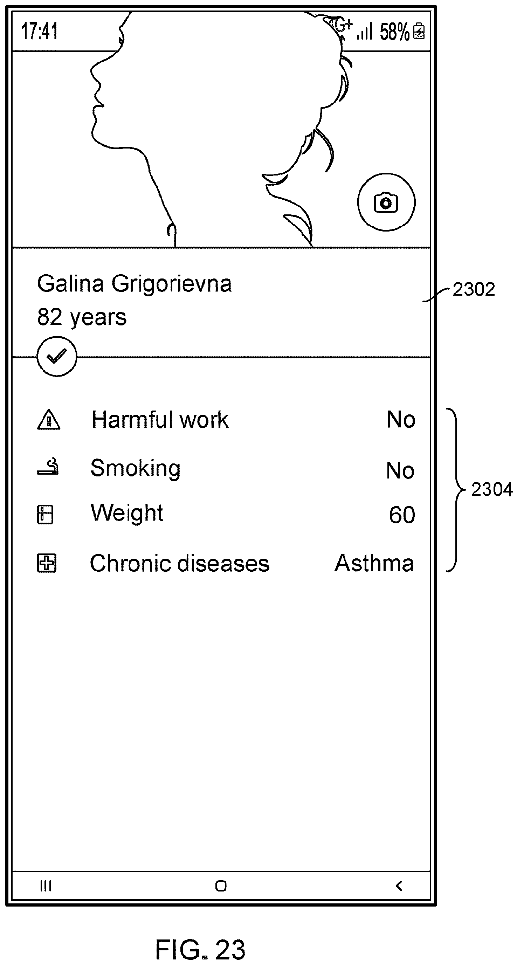

Figure 23 shows a screen that identifies the subject 2302 and provides information 2304 about factors related to respiratory conditions.

In some implementations, a stethoscope attachment for a smart phone is used to detect the respiratory sounds and generate the audio signal. If a stethoscope attachment is used, a Bluetooth connection is not effected, and the smartphone built-in microphone is responsible for detecting the respiratory sounds and generating the audio signal; the stethoscope attachment directs the sound into the smart phone microphone through a cone. Along with recording a signal, the patient can complete a periodic questionnaire or diagnostic questionnaire, or both, which are available in the user interface of the mobile application. The questionnaires are designed to help monitor or determine the respiratory condition of health, because similar respiratory sounds may be characteristic of different diseases or other respiratory conditions, so it may be necessary to ask additional questions in order to determine the actual respiratory condition more accurately.

In some examples, the questionnaires contain questions about general health, the presence or absence of pathological symptoms, and quantitative characteristics, such as temperature or ESR (erythrocyte sedimentation rate). The answers to the periodic or diagnostic health questionnaire's questions and the files of recorded respiratory sound audio signals are then sent to the remote server for processing in order to obtain a classification assessment of the class of respiratory sound, determine the corresponding respiratory condition, provide a recommendation, and save the collected data to maintain the case history for the subject.

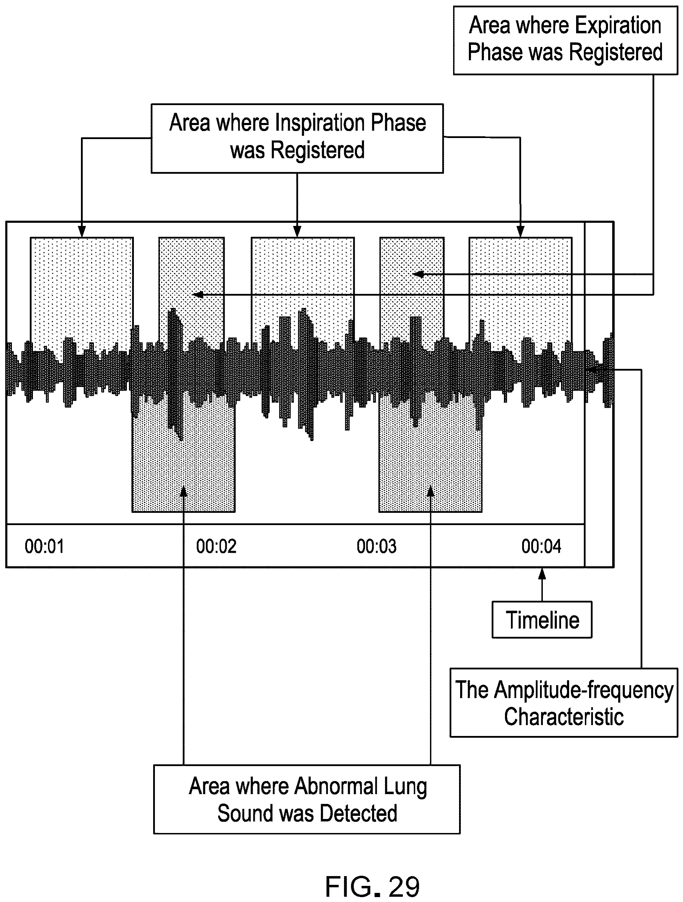

In some implementations, processing of the audio signals includes several stages:
1. The stage of audio signal pre-processing, namely, noise suppression and frequency-time representation charting, in particular, the calculation of Mel spectrum and Mel frequency cepstral coefficients and their representation in an image file, which is useful to prepare the input audio signals for the machine learning algorithm and to augment the pre-processed data.
2. Although not always required and therefore optional, the step of determining the respiration phases (a figure of the Eurasia patent application shows an example of the algorithm) includes marking the amplitude-time characteristic according to the phases of breathing (inspiration and expiration) and wheezing (if wheezing occurred) detected at these phases. This is carried out, for example, in the following way:
   a. The original audio signal is obtained in a pulse-code modulation form (amplitude-time representation). The assumption is that depressions on the waveform correspond to pauses between inspirations and expirations.
   b. The original audio signal is compressed by several orders of magnitude (from 212,000 points to about 2,500 points).
   c. The resulting compressed audio signal is approximated, with small depressions smoothed and removed.
   d. Then all the depressions that are below a threshold value are marked (for example, 0.02 quantile, i.e., only 2% of the points on the diagram).
   e. Short segments and phases are removed, including everything which is shorter than 30% of the average width of the segments and phases.
   f. The remaining intervals are considered respiratory phases based on the assumption that the inspiration is followed by expiration.
   The approximation algorithms are widely known; the result of steps a through f is illustrated in Figure 29 with a mark-up, which allows highlighting the areas on the timeline where inspiration, expiration, and wheezing were detected. The amplitude-frequency characteristic of the recorded sounds is also shown.
3. The stage of classification assessment (determining the sound classes) of the respiratory sound using neural network machine learning algorithms, which results in the probability of correctly assigning the respiratory sound to, for example, one of the wheezing classes or healthy breathing class (no wheezing).
4. The stage of operation of the expert system using the results of the classification and received answers to the periodic questionnaire. The expert system is designed to give a final assessment of the patient's condition of health or other respiratory condition, taking into account the automatic classification of respiratory sound and the questionnaire answers, and possibly other things.

A non-limiting example of the implementation in the respiratory system technology is given below.

### Example:

A patient with a clinical diagnosis of COPD answers a diagnostic questionnaire:
We learn the following about the patient from their answers:
   - the disease began with coughing
   - temperature is not elevated
   - coughing is wet, persistent, and has lasted for several years
   - there is much sputum
   - dyspnea sometimes happens
   - there is no weakness
   - there is no chest pain
   - there are no chills or perspiration
   - there are stomach problems
   - no blood test was made over the last few days
   - has been smoking, for a long time
   - is allergic to pollen and dust
Next, the patient undergoes auscultation.

The expert system's inputs receive answers from the questionnaire, signal data from the auscultation, and information from the patient's profile, and the expert system can take into account the seasonality of diseases. For example, exacerbations of allergic asthma are characteristic of the spring-summer period, while pneumonia and COPD exacerbations are characteristic of the wet season. The patient indicates their chronic diseases in their profile in the mobile application, so that the system can take into account the expected problems; for example, if it is the wet season and the patient has COPD in their profile, and crackles are detected, this will signal the need for an urgent visit to a doctor. The expert system performs the analysis for each disease or other respiratory condition (the probability of having each disease individually). Based on the questionnaire responses and auscultation (rhonchi found in some parts of the lungs, for example), the expert system determines that there is a high probability of COPD.

After that, the results of the analysis of audio signals and the answers to the periodic questionnaire are available to both the patient and the attending doctor. The patient receives a recommendation about his current health status or other respiratory condition in the user interface of the mobile application (the recommendation is automatically generated on the server and is sent from it to the patient's mobile application) and can also monitor the dynamics of improvement or deterioration of their respiratory condition.

Over time, the statistics need to be replenished, and for this purpose the patient can complete new questionnaires (for example, for COPD) and perform or be subjected to auscultations, and will receive updated statistics. The doctor receives the detailed information on the patient's respiratory condition, as well, and is additionally able to listen to the respiratory sound audio signals, if there are doubts about the correctness of the results of respiratory sound automatic classification or visual marking or both. After the patient sends data to the server from the mobile application, the data is saved in a database of the server, where it becomes available to an authorized doctor from the website and to the patient who can listen to them in the mobile application and view the results of the automatic classification of respiratory sound. Once the above procedure is done for the first time, the doctor and the patient can contact each other on their mutual initiative.

Among other benefits, a practical significance of the respiratory system technology described here is in providing an affordable and cheap method of automatic detection, screening, monitoring, and diagnosing of respiratory diseases and other respiratory conditions. On the one hand, the resulting information can be used by the patient at home, which is intended to improve the quality of life of people with respiratory diseases and respiratory conditions, and particular chronic diseases, due to early detection of the impairment of lung health. On the other hand, the respiratory condition technology described here is designed to reduce the duration of admission of patients at medical institutions and the costs of patient care due to the available history of the respiratory condition, prevention of the cases of unreasonable visits or, contrariwise, increase in the number of timely therapeutic interventions prior to the disease exacerbation.

The respiratory system technology provides a hardware and software solution for home use, and achieves high accuracy of the algorithm for automatic determination of the respiratory sound class (about 80%), which, combined with medical examination, makes it possible to virtually eliminate human error in the diagnosis or other management of respiratory disease or other respiratory conditions.

The algorithm for automatic determination of the respiratory sound class adapts known approaches in machine learning to the task of classifying respiratory sounds; the adaptation is justified by the limited size (in terms of labeled signals) of the respiratory sound audio signals database and their specificity, and involves the use of the appropriate neural network architecture and learning transfer approach in the learning process.

In addition to the implementations described in the Eurasian patent application and others discussed above, the following implementations, and combinations of them, could also be part of the respiratory condition technology.

In some implementations, the classification model can use a SqueezeNET-based architecture (https://en.wikipedia.org/wiki/SqueezeNet) to reduce the size of the neural network and make the operation of the model faster. In such implementations, the input can be Mel spectrograms using less information. For example, for pre-processing and classification of a segment of a sound record, the pre-processing and classification can be achieved within the timeframe of about 80 ms and the size of the file can be in the order of 2.85 Mb.

In some implementations, marking of the lung sound record as presented to a doctor, a patient, or another interested party, can highlight exact areas in which abnormal sound was detected. For this purpose, the respiratory sound record can be split into segments each of which is separately classified as to the class of respiratory condition. For example, a first segment of a record could be classified as rhonchi while the subsequent, second segment could be classified as crackles. When the results of the classification are presented through a user interface, the viewer can be given more information about respiratory sound classes associated with the recorded sound, which can enable the viewer to understand exactly when each classified sound was detected.

As mentioned earlier, in some implementations, the classification model can be executed as part of an application running on a mobile device, such as an Android smartphone. This can be possible if the memory space taken up by the classification model is small enough and the processing resources required for executing the model are sufficiently small. In addition, running the classification model on a mobile device can be made possible by using quantization techniques, deleting unused constants, and other conventional adjustments including operations in Tensorflow utilities.

In some implementations, the trained machine language (classification) model may be implemented on ARM M-series microcontrollers to form standalone hardware and firmware devices which could have a wide variety of uses in medical devices, stethoscopes, and other equipment. For this purpose, the trained machine language (classification) model can be parsed to extract operation graphs and weights; C++ source code files can be generated including one file containing only quantized weights as static constants, a second file containing configuration information for an ANN (artificial neural network) such as layer sizes and dimensions, and a third file containing audio processing code and chained ANN layer calls from the CMSIS-NN library available from ARM (https://community.arm.com/developer/ip-products/processors/b/processors-ip-blog/posts/new-neural-network-kernels-boost-efficiency-in-microcontrollers-by-5x).

As mentioned earlier, in some cases, the use of the detection algorithm to identify separately the inspiration and expiration portions of a sound record can be disregarded for purposes of the implementation of the mark-up highlighting areas where abnormal sounds are detected. In some implementations, detection of inspiration and expiration portions of the sound record can be useful for bronchial breath detection, although it may be better to use machine learning techniques for this purpose.

In some examples, the classification of sound records into respiratory sound classes can be achieved at an accuracy level of 93% across the following classes: wheeze plus rhonchi, fine crackles plus coarse crackles, normal condition, and artifacts. The artifacts could include, for example, heart sounds, electromagnetic and environmental interference, and stethoscope membrane rubbing against the skin.

In some implementations, instead of using machine learning classification models, a non-machine learning algorithm can be developed based on frequency filtering and dominant frequency detection. This may be useful in contexts in which it may not be possible to create data sets clean enough for machine learning techniques to be applied to typical classes determined by human hearing. Among other reasons, this is because of significant mid-frequency abnormal sounds that may be present in wheezes and rhonchi classes. In a non-machine learning context, the data set can be cleaner and can be used as a differentiator. Artificial neural network (ANN) classification could be used to classify wheezing, for example, and then the differentiating algorithm can indicate whether the wheezing is at a high frequency, a mid frequency, or low frequency.

In some implementations, it may be possible to generate a classification model capable of multiclass classification configured to differentiate between fine crackles and coarse crackles, in other words in cases of sound records that contain mixed sounds, such as rhonchi combined with crackles during the same sound record.

In some cases, the machine learning classification model can be based on two stages: data set pre-processing and model training. For the first stage artificial data can be generated based on natural data, for example by recording normal breathing sounds, cutting a wheezing sound from a recording of wheezing, augmenting the wheezing and adding it to the normal breathing sound automatically. Rectangles can then be added to the resulting spectrograms identifying abnormal sounds in the sound records. The resulting data set will then contain spectrograms and rectangle metadata identifying the exact portions of the spectrograms containing abnormal sounds. For the second stage, a single shot multi box detector (SSD) can be trained and used in mobile device applications.

Some implementations of the respiratory condition technology can be applied to the following use cases as examples.

Case 1. A child is coughing but the meaning of the coughing is unclear. The parents are worried about the possibility of pneumonia and want to know whether to take the child to the doctor. The parents record the child's lung sounds which are analysed and result in a presentation on the user interface of a mobile device of the parents. The presentation can include an indication of abnormal sounds present in the recording, typical respiratory conditions associated with those sounds (for example, according to scholarly literature), and the recommendation about whether to take the child to the doctor.

Case 2. Anxious smokers may want to know whether they may have developed lung cancer, COPD, or other adverse respiratory conditions associated with smoking.

Case 3. Health-conscious or technology savvy people in the 45- to 65-year-old age range may have a continuing interest in their respiratory condition.

Case 4. People living in polluted areas may be susceptible to frequent bronchitis and colds and would like to be able to track their progress and identify their presence.

In various implementations, the sound sensor can be a variety of commercially available hardware to record lung sounds at an adequate quality level, such as the Littmann classic stethoscope plus a eKuore attachment, or custom-made wired stethoscopes, mass-produced Bluetooth capable stethoscopes, or smart phones such as iPhones or Android devices running on version 8+ or later. In some cases, the sound sensor could be embedded in a shirt or other garment.

In some implementations of the user interface on the mobile device, the user interface guides a subject through the process of capturing the sound recording and then displays results through the user interface. The results can be shared using a link.

In some cases, early signs of exacerbations of types of chronic lung disease can be determined from the sound records and series of the sound records. It may be possible to detect crackles in the recorded sounds that are indicative of exacerbations of the chronic lung disease that are imminent (for example, within a few days). The characteristics of the recorded sounds that may be correlated with the exacerbations can include the class of lung sound, the location within the subject, and the intensity. In some cases, a combination of the change in recorded sounds and a change in symptoms may be most effective in predicting an exacerbation and provide the least exacerbation to the subject. The determination of the correlation between sounds, symptoms, and expected future exasperation can be based on collections of samples of recorded sounds and labeling of those sounds by doctors.

Case 5. A patient having COPD is hospitalized because of an exacerbation of the symptoms. The nurse in the hospital issues her a recording device for capturing sound recordings, installs an application on the patient's mobile device, and educates the patient about its benefits and use. The patient takes home the equipment and self-monitors daily during a 1- to 2-month period to detect early signs of a subsequent exacerbation. When a predicted subsequent exacerbation is detected, the patient is alerted and may either be referred for therapy or go to a doctor. The determinations for such a patient can be sent automatically through the Internet into existing computer systems of a medical care provider, for example.

Some implementations of the mobile device application and its user interface to be used by a subject or other interested party are designed for self-management of a chronic or other respiratory condition. The application can include features to enable the user to easily log in symptoms being experienced from time to time, to enable the user to capture sound recordings and to teach the user how to do so, to display the development and progress of a chronic respiratory condition in charts and through other user interface techniques, and provide a mechanism for contacting a medical care provider or other assistant.

In some applications, the respiratory condition technology can serve as a second validation source in connection with diagnosis or interpretation of a respiratory condition.

Case 5. People with asthma may use their peak flow meters or smart inhalers to alert them of impending exacerbations of their respiratory condition, but those techniques may not be precise. The respiratory condition technology can be used to supplement the information provided by peak flow meters or smart inhalers to more accurately predict or validate an impending exacerbation and reduce false positives and false negatives. In some implementations, the mobile device application of the respiratory condition technology, which might normally use its own peak flow information and display related plots, could accept this information from separate smart peak flow meters.

Case 6. Cystic fibrosis (CF) patients, because they are born with CF and need uninterrupted treatment, are experts on their own health. Yet such patients can benefit from a separate source of objective warnings about their disease, which can help with decisions on the level of therapy and assessments of their regular physical rehabilitation techniques. The respiratory condition technology can provide a source of such assessments in conjunction with other sources of information.

Case 7. In some cases it may be possible not only to provide diagnosis, information, or suggestions about respiratory conditions, but also make suggestions about therapies using the respiratory condition technology. Such suggestions may relate to therapy tracking, exacerbation prediction as mentioned above, medication reminders, or air pollution data. For example, information may be provided to a patient suggesting when to take COPD antibiotics during exacerbation and when use of merely steroids would be sufficient. In some instances, the respiratory condition technology can assess the reaction of a patient to a therapy and may use correlation analysis of population data to suggest which therapy would be best for a given time period.

In some implementations, the respiratory condition technology not only accumulates, analyses, and reports on respiratory conditions derived from sound recordings of individuals, but also can analyse and report on aggregated and statistical information derived from populations of users. For example, information can be accumulated and analysed based on demographics, geography, and other factors. In some cases, maps can be generated and provided for display through user interfaces of mobile devices to illustrate the prevalence, severity, or history of one or more respiratory conditions across a nation or a political subdivision or a multi-national region. For example, information about the progression of upper respiratory tract infections (URTIs) across a geographic region could be illustrated by visualization techniques on a displayed map.

In some instances, the results of statistical analyses could be reported to users. For example, the appearance in the database of an abundance of normal recordings (presumably captured by healthy individuals who started coughing due to URTIs) could suggest a threat to COPD patients (because half of URTIs contracted by COPD patients produce exacerbation) that could be reported to them through the user interfaces. In another example, if a large number of asthmatic patients experience exacerbation after visiting a certain place, other asthmatic patients can be alerted.

Among other respiratory diseases that could be screened, monitored, and diagnosed using the respiratory condition technology are IPF (idiopathic pulmonary fibrosis), which might be subjected to broad screening of large populations at public locations; cancer (based on failure of previously successful therapies for COPD); combined cardiovascular and respiratory conditions determined by analysis of heart sounds and respiratory sounds followed by intervention to reduce severity; changes in respiratory sounds that suggest alerting the user to do a more precise measurement; and screening, monitoring, and diagnosing lung transplant patients to detect lower lung infections at an early stage.

Other implementations are also within the scope of the claims below.

### EMBODIMENTS:

Although the present invention is defined in the claims, it should be understood that the present invention can also (alternatively) be defined in accordance with the following embodiments:
1. A machine-based method comprising
   receiving a sound record representing respiratory sounds of a subject acquired by auscultation,
   by machine, transforming the received sound record into a time-frequency domain graphical representation,
   by machine, applying the time-frequency domain graphical representation to a classifier model to determine a sound class for the respiratory sounds of the subject, and
   by machine, inferring a respiratory condition of the subject based at least on the sound class determined by the classifier model.
2. The method of embodiment 1 in which the time-frequency domain graphical representation comprises a Mel spectrogram.
3. The method of embodiment 2 in which the time-frequency domain graphical representation comprises a color Mel spectrogram.
4. The method of embodiment 1 in which the classifier model comprises a neural network model.
5. The method of embodiment 1 comprising using an expert system for inferring the respiratory condition of the subject based at least on the sound class determined by the classifier model.
6. The method of embodiment 5 in which the expert system infers the respiratory condition of the subject based also on other information about the subject.
7. The method of embodiment 6 in which the other information about the subject is received from the subject in response to a questionnaire.
8. The method of embodiment 6 in which the other information about the subject comprises demographic information.
9. The method of embodiment 6 in which the other information about the subject comprises information about a respiratory condition.
10. The method of embodiment 1 comprising presenting information about the inferred respiratory condition through a user interface of a device.
11. The method of embodiment 10 in which the information presented through the user interface comprises a graphical representation of the sound record during the period of time.
12. The method of embodiment 11 in which the graphical representation of the sound record is color-coded according to sound class.
13. The method of embodiment 10 in which the information about the inferred respiratory condition presented through the user interface comprises information about management of a respiratory condition.
14. The method of embodiment 1 comprising receiving multiple sound records taken at different sound capture points on the subject.
15. The method of embodiment 14 in which the sound capture points are determined algorithmically based on the respiratory condition, and are presented to the subject through a user interface of a mobile device.
16. The method of embodiment 1 comprising receiving multiple sound records taken at a particular sound capture point on the subject.
17. The method of embodiment 16 comprising, by machine, performing a principal component analysis or other correlational analysis or multidimensional analysis on the multiple sound records.
18. The method of embodiment 1 in which the sound record has degraded quality.
19. The method of embodiment 18 in which the graded quality is based on noise or improper auscultation or a combination of them.
20. A machine-based method comprising
   receiving a first number of sound records, each of the sound records representing respiratory sounds of a subject acquired by auscultation, each of the sound records having known sound classes determined by one or more experts,
   pre-training initial convolutional layers of a neural network using a second number of known spectrograms not necessarily related to sound records,
   after the pre-training, training the initial convolutional layers of the neural network using the first number of sound records and the known sound classes,
   the second number of sound records being at least an order of magnitude larger than the first number of sound records,
   receiving a sound record for which of the sound class has not been determined,
   applying the received sound record to the neural network to determine a sound class for the sound record.
21. The method of embodiment 20 comprising enhancing operation of the neural network by one or more of the following: detecting and eliminating artifacts in the sound records, differentiating different classes of sound records, or adding new sound classes based on new sound records having known sound classes determined by the one or more experts.
22. The method of embodiment 20 in which the neural network comprises a truncated model.
23. The method of embodiment 22 in which the truncated model comprises a SqueezeNET model.
24. The method of embodiment 22 in which the truncated model is executed on a mobile device.
25. The method of embodiment 22 in which the truncated model is executed on an ARM processor.
26. The method of embodiment 20 comprising executing an expert system using the determined sound class for the sound record to infer a respiratory condition of the subject.
27. The method of embodiment 26 comprising presenting information about the inferred respiratory condition through a user interface of the device.
28. The method of embodiment 20 in which the applying of the received sound record to the neural network to determine a sound class for the sound record is performed at a server remote from a location where the sound record is captured.
29. The method of embodiment 20 in which the applying of the received sound record to the neural network to determine a sound class for the sound record is performed at a mobile device.
30. The method of embodiment 20 in which the applying of the received sound record to the neural network to determine a sound class for the sound record is performed at a combination of a mobile device and a server remote from the mobile device.
31. The method of embodiment 20 in which the applying of the received sound record to the neural network to determine a sound class for the sound record comprises generating a Mel spectrogram for the received sound record.
32. The method of embodiment 20 in which the applying of the received sound records of the neural network to determine a sound class for the sound record comprises determining a key-value pair for each of the sound records in which the key comprises the sound capture point on the subject and the value comprises the sound class.
33. The method of embodiment 20 in which the sound class comprises at least one of: normal sound, wheezes, rhonchi, fine crackles, coarse crackles, skin rubbing artifacts, interference artifacts, and heartbeat artifacts.
34. A machine-based method comprising
   receiving from an application running on a mobile device of a subject information related to one or more respiratory conditions of the subject, the information including respiratory sounds captured from the subject by auscultation,
   processing the information at a server, and
   presenting to a healthcare provider through a user interface of a device, the information received from the application running on the mobile device related to the one or more respiratory conditions of the subject, and
   receiving at the server from the healthcare provider a determination about managing the one or more respiratory conditions.
35. The method of embodiment 34 in which the information received from the application running on the mobile device comprises information entered by the subject through a user interface on the mobile device.
36. The method of embodiment 34 in which the processing of the information at the server comprises applying the respiratory sounds to a classification model to determine sound classes for the respiratory sounds.
37. The method of embodiment 34 in which the processing of the information at the server comprises inferring one or more respiratory conditions of the subject.
38. The method of embodiment 37 in which the inferring of the one or more respiratory conditions of the subject is based on the respiratory sounds and on other information received from the subject through the mobile device.
39. The method of embodiment 34 comprising presenting the determination of the healthcare provider about managing the one or more respiratory conditions to the subject through the mobile device.
40. The method of embodiment 34 in which the determination about managing the one or more respiratory conditions comprises one or more of a diagnosis, a prescription of therapy, training, guidance, or questions.
41. The method of embodiment 34 in which the determination about managing the one or more respiratory conditions comprises a binary determination, and the method comprising presenting the binary determination to subject through mobile device.
42. The method of embodiment 41 in which the binary determination presented to the subject comprises a determination that the respiratory condition is dangerous or not dangerous, or that the subject should see a doctor or need not see a doctor.
43. A machine-based method comprising
   receiving from a device of a subject answers to one or more questions about the subject, and
   at a server, applying the answers to an expert system to infer a respiratory condition of the subject, the expert system inferring the respiratory condition of the subject based also on sound records captured by auscultation of the subject.
44. The method of embodiment 43 in which the questions are part of a diagnostic questionnaire or periodic questionnaire.
45. The method of embodiment 44 in which the diagnostic questionnaire or periodic questionnaire relates to a particular respiratory condition.
46. The method of embodiment 44 in which the sound records captured by auscultation of the subject are also received from the device of the subject.
47. A machine-based method comprising
   receiving at a mobile device of a subject, sound records captured by auscultation at one or more sound capture points on the subject, the sound records being captured at successive times over a period of time,
   based on the sound records captured at the successive times, inferring changes in a respiratory condition of the subject, and
   presenting information about the changes in the respiratory condition of the subject through the mobile device.
48. The method of embodiment 47 in which the inferring of changes in the respiratory condition of the subject comprises inferring the respiratory condition of the subject at each of the successive times and comparing the inferred respiratory conditions.
49. The method of embodiment 48 in which the inferring of the respiratory condition of the subject at each of the successive times comprises classifying at least one of the sound records as representing one or more sound classes.
50. The method of embodiment 49 in which the inferring of the respiratory condition of the subject at each of the successive times comprises applying an expert system to the one or more sound classes.
51. The method of embodiment 47 in which the inferring of the changes in the respiratory condition are performed at least in part at the mobile device.
52. The method of embodiment 47 in which the inferring of the changes in the respiratory condition are performed at least in part at a server.
53. The method of embodiment 47 in which the respiratory condition comprises a chronic respiratory condition.
54. The method of embodiment 53 in which the chronic respiratory condition comprises COPD.
55. The method of embodiment 47 in which the inferred changes in the respiratory condition of the subject comprise exacerbations.
56. The method of embodiment 47 comprising presenting information about the changes in the respiratory condition of the subject to a healthcare provider through a user interface of a device.

## Claims

1. A system comprising one or more processors (210, 212, 260) executing a machine learning neural network classifier model (216), wherein the machine learning neural network classifier model (216) is formed by a machine-based, transfer learning method comprising
receiving a first number of sound records, each of the sound records representing respiratory sounds of a subject acquired by auscultation, each of the sound records having known sound classes determined by one or more experts, wherein the sound classes comprises at least one of normal sound, wheezes, rhonchi, fine crackles, coarse crackles, skin rubbing artifacts, interference artifacts, or heartbeat artifacts,
pre-training initial convolutional layers of a neural network as a feature extractor using a second number of known images not related to sound records,
after the pre-training, training the neural network using the first number of sound records and the known sound classes,
the second number of images being at least an order of magnitude larger than the first number of sound records.
the one or more processors configured to:
receive a sound record representing respiratory sounds of a subject acquired by auscultation,
transform the received sound record into a time-frequency domain graphical representation that comprises a Mel spectrogram,
determine a sound class for the respiratory sounds of the subject by applying the time-frequency domain graphical representation to the machine learning neural network classifier model (216), and
infer a respiratory condition of the subject based at least on the sound class determined by the machine learning neural network classifier model.

2. The system of claim 1 in which the time-frequency domain graphical representation comprises a color Mel spectrogram.

3. The system of claim 1 comprising using an expert system (218) for inferring the respiratory condition of the subject based at least on the sound class determined by the machine learning neural network classifier model.

4. The system of claim 3 in which the expert system infers the respiratory condition of the subject based also on other information about the subject, for example, in which the other information about the subject is received from the subject in response to a questionnaire.

5. The system of claim 4 in which the other information about the subject comprises demographic information or information about a respiratory condition.

6. The method of claim 1 comprising presenting information about the inferred respiratory condition through a user interface of a device.

7. The system of claim 1 in which the information presented through the user interface comprises a graphical representation of the sound record during the period of time, and optionally wherein the graphical representation of the sound record is color-coded according to sound class.

8. The system of claim 1 in which the information about the inferred respiratory condition presented through the user interface comprises information about management of a respiratory condition.

9. The system of claim 1 in which the one or more processors are configured to receiving multiple sound records (222) taken at different sound capture points on the subject and
optionally wherein the sound capture points are determined algorithmically based on the respiratory condition, and are presented to the subject through a user interface of a mobile device.

10. The system of claim 1 in which the one or more processors are configured to receive multiple sound records (222) taken at a particular sound capture point on the subject, and
optionally wherein the system is further configured to perform a principal component analysis or other correlational analysis or multidimensional analysis on the multiple sound records.

11. The system of claim 1 in which the method comprises enhancing operation of the neural network by one or more of the following: detecting and eliminating artifacts in the sound records, differentiating different classes of sound records, or adding new sound classes based on new sound records having known sound classes determined by the one or more experts.

12. The system of claim 1 in which the neural network comprises a truncated model, for example, in which the truncated model comprises a SqueezeNET model.

13. The system of claim 12 in which the truncated model is executed on a mobile device or on an ARM processor.

14. A machine-based method performed by one or more processors (210, 212, 260) executing a machine learning neural network classifier model (216), wherein the machine learning neural network classifier model (216) is formed by a machine-based, transfer learning method comprising
receiving a first number of sound records, each of the sound records representing respiratory sounds of a subject acquired by auscultation, each of the sound records having known sound classes determined by one or more experts, wherein the sound classes comprises at least one of normal sound, wheezes, rhonchi, fine crackles, coarse crackles, skin rubbing artifacts, interference artifacts, or heartbeat artifacts,
pre-training initial convolutional layers of a neural network as a feature extractor using a second number of known images not related to sound records,
after the pre-training, training the neural network using the first number of sound records and the known sound classes,
the second number of images being at least an order of magnitude larger than the first number of sound records
wherein the machine-based method that is performed by the one or more processors (210, 212, 260) executing the machine learning neural network classifier model comprises
receiving a sound record representing respiratory sounds of a subject acquired by auscultation,
transforming the received sound record into a time-frequency domain graphical representation that comprises a Mel spectrogram,
determining a sound class for the respiratory sounds of the subject by applying the time-frequency domain graphical representation to the machine learning neural network classifier model (216),
wherein the sound class for the respiratory sounds of the subject is suitable for inferring a respiratory condition of the subject.

15. The method of claim 14 in which the time-frequency domain graphical representation comprises a color Mel spectrogram.

16. The method of claim 14 comprising receiving multiple sound records (222) taken at different sound capture points on the subject and
optionally wherein the sound capture points are determined algorithmically based on the respiratory condition, and are presented to the subject through a user interface of a mobile device.

17. The method of claim 14 comprising receiving multiple sound records (222) taken at a particular sound capture point on the subject, and
optionally wherein the method further comprises performing a principal component analysis or other correlational analysis or multidimensional analysis on the multiple sound records.
